(19) Europäisches Patentamt
European Patent Office
Office européen des brevets

(11) EP 4 125 780 B1

(12) # EUROPÄISCHE PATENTSCHRIFT

(45) Veröffentlichungstag und Bekanntmachung des Hinweises auf die Patenterteilung:
**13.05.2026 Patentblatt 2026/20**

(21) Anmeldenummer: **21704715.8**

(22) Anmeldetag: **01.02.2021**

(51) Internationale Patentklassifikation (IPC):
***A61K 8/22*** ***(2006.01)*** ***A61K 8/898*** ***(2006.01)***
***A61Q 5/06*** ***(2006.01)***

(52) Gemeinsame Patentklassifikation (CPC):
**A61Q 5/065; A61K 8/22; A61K 8/898**

(86) Internationale Anmeldenummer:
**PCT/EP2021/052236**

(87) Internationale Veröffentlichungsnummer:
**WO 2021/190810 (30.09.2021 Gazette 2021/39)**

(54) **VERBESSERUNG DER WASCHECHTHEIT VON PIGMENTHALTIGEN FÄRBEMITTELN DURCH ANWENDUNG EINES OXIDATIVEN VORBEHANDLUNGSMITTELS**

IMPROVING THE FASTNESS TO WASHING OF PIGMENT-CONTAINING DYES BY USING AN OXIDATIVE PRETREATMENT AGENT

AMÉLIORATION DE LA SOLIDITÉ AU LAVAGE DE COLORANTS CONTENANT DES PIGMENTS À L’AIDE D’UN AGENT DE PRÉTRAITEMENT OXYDANT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.03.2020 DE 102020203743**

(43) Veröffentlichungstag der Anmeldung:
**08.02.2023 Patentblatt 2023/06**

(73) Patentinhaber: **Henkel AG & Co. KGaA**
**40589 Düsseldorf (DE)**

(72) Erfinder:
- **KRUCK, Constanze**
  **41515 Grevenbroich (DE)**
- **HILBIG, Sandra**
  **44894 Bochum (DE)**
- **MOCH, Melanie**
  **41542 Dormagen (DE)**
- **KESSLER-BECKER, Daniela**
  **51371 Leverkusen (DE)**

(56) Entgegenhaltungen:
**WO-A1-2017/108828** **DE-A1- 102014 218 006**
**US-A1- 2013 149 358**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Gegenstand der vorliegenden Anmeldung ist ein kosmetisches Verfahren zum Färben von keratinischen Fasern, insbesondere menschlichen Haaren, welches die Anwendung von mindestens zwei verschiedenen Mitteln (V) und (F) umfasst. Das Mittel (V) stellt ein Vorbehandlungsmittel dar, welches in einem kosmetischen Träger mindestens ein Oxidationsmittel enthält. Das Färbemittel (F) enthält in einem kosmetischen Träger mindestens ein aminofunktionalisiertes Silikonpolymer (F-1) und mindestens ein Pigment (F-2).

Die Veränderung von Form und Farbe von keratinischem Material, insbesondere von menschlichen Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr langanhaltende Färbeergebnisse aus.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

Für kurzzeitige Farbveränderungen auf dem Haar und/oder der Haut ist der Einsatz von Farbpigmenten bekannt. Unter Farbpigmenten werden im Allgemeinen unlösliche, farbgebende Substanzen verstanden. Diese liegen ungelöst in Form kleiner Partikel in der Färbeformulierung vor und lagern sich lediglich von außen auf den Haarfasern und/oder der Hautoberfläche ab. Daher lassen sie sich in der Regel durch einige Wäschen mit tensidhaltigen Reinigungsmitteln wieder rückstandslos entfernen. Unter dem Namen Haar-Mascara sind verschiedene Produkte dieses Typs auf dem Markt erhältlich.

WO 2017/108828 A1 betrifft Mittel zum Färben von Keratinfasern, welche mindestens einen Triaylmethanfarbstoff, ein Aminosilikon und ein Tensid enthalten.

US 2013/149358 A1 beschreibt ein Verfahren zur Erzeugung langanhaltender Färbungen auf Keratinsubstraten, welches die Anwendung eines bestimmten Aminosilikons und eines nichtsphärischen, partikelförmigen Materials umfasst, wobei letzteres auch ein Pigment sein kann. Gegenstand von DE 10 2014 218006 A1 ist eine Verpackungseinheit zur Färbung von Keratinfasern, umfassend einen ersten Container mit einem ersten Mittel und einen zweiten Container mit einem zweiten Mittel. Das erste Mittel enthält ein Aminosilikon und einen direktziehenden Farbstoff, und das zweite Mittel beinhaltet eine bestimmte Menge eines Oxidationsmittels.

Wünscht sich der Anwender besonders langanhaltende Färbungen, so ist die Verwendung von oxidativen Färbemitteln bislang seine einzige Option. Doch trotz vielfacher Optimierungsversuche lässt sich bei der oxidativen Haarfärbung ein unangenehmer Ammoniakgeruch bzw. Amingeruch nicht vollständig vermeiden. Auch die mit dem Einsatz der oxidativen Färbemittel nach wie vor verbundene Haarschädigung wirkt sich auf das Haar des Anwenders nachteilig aus. Eine nach wie vor bestehende Herausforderung ist daher die Suche nach alternativen, leistungsstarken Färbeverfahren. Ein mögliches, alternatives Färbesystem, das in letzter Zeit zunehmend in den Fokus rückt, beruht auf dem Einsatz von farbigen Pigmenten.

Die Färbung mit Pigmenten bietet verschiedene wesentliche Vorteile. Da die Pigmente sich lediglich von außen an die Keratinmaterialen, insbesondere an die Haarfasern, anlagern, lassen sich nicht mehr erwünschte Färbungen schnell und einfach rückstandslos entfernen und bieten dem Anwender auf diese Weise die Möglichkeit, unmittelbar und ohne großen Aufwand zu seiner Ursprungshaarfarbe zurückkehren zu können. Insbesondere für die Konsumenten, die ihre Haare nicht regelmäßig nachfärben möchten, ist dieser Färbeprozess daher besonders attraktiv.

In aktuellen Arbeiten wurde das Problem der geringen Haltbarkeit dieses Färbesystems adressiert. In diesem Zusammenhang konnte gefunden werden, dass die Waschechtheit der mit Pigmenten erhaltenen Farbresultate durch Kombination der Pigmente mit bestimmten aminofunktionalisierten Silikonpolymeren stark verbessert werden konnte. Darüber hinaus konnte durch die Wahl besonders gut geeigneter Pigmente und Pigmentkonzentrationen auf dunklem Haar ein helleres Farbergebnis erzielt werden, so dass mit diesem Färbesystem sogar eine Aufhellung möglich wurde, die bis dato ausschließlich mit oxidativen Haarbehandlungsmitteln (Bleich- bzw. Blondiermitteln) möglich war.

Neben diesen vielen Vorteilen besitzt das auf Pigmenten basierende Färbesystem jedoch immer noch einige Nachteile, die in der geringen Eindringtiefe der Pigmente in das keratinische Material begründet sind. Da die Pigmente nicht in das Keratinmaterial bzw. die Keratinfaser hinein diffundieren, sondern sich lediglich in Form einer Hülle bzw. eines Films an der Außenseite des Keratinmaterials ablagern, ist die Waschechtheit der mit diesem System erzeugten Färbungen nach wie vor verbesserungsbedürftig. Auch wenn durch Auswahl besonders gut geeigneter Pigmente bzw. Aminosilikone schon eine gute Verbesserung der Waschechtheit erzielt werden konnte, ist diese immer noch nicht als optimal zu bezeichnen.

Es war daher die Aufgabe der vorliegenden Anmeldung, ein auf Pigmenten basierendes Färbeverfahren

aufzufinden, mit dem sich intensive Färbungen mit verbesserter Waschechtheit erzielen lassen. Hierbei sollten die mit diesem Verfahren gefärbten Keratinmatieralien bzw. insbesondere Haare unabhängig vom Haartyp und von anderen zuvor angewendeten kosmetischen Produkten Färbungen in genau der Nuance und Intensität liefern, die der Anwender nach Lekture der entsprechenden Verpackungsinformation bzw. Gebrauchsanweisung auch erwartet. Auch nach einem längeren Zeitraum und mehreren Haarwäschen sollten bei den gefärbten Haaren keine unerwünschte Nuancenverschiebungen auftreten.

Überraschenderweise hat sich nun herausgestellt, dass die Waschechtheit von Pigment-Färbesystemen stark verbessert werden kann, wenn keratinische Materialien, insbesondere Haare, vor dem eigentlichen Färbevorgang einer oxidativen Vorbehandlung unterzogen werden.

Ein erster Gegenstand der vorliegenden Erfindung ist ein Verfahren zum Färben von keratinischen Fasern, insbesondere menschlichen Haaren, umfassend die folgenden Schritte in der angegebenen Reihenfolge:

(1) Applizieren eines Vorbehandlungsmittels (V) auf die keratinischen Fasern,
(2) Einwirken des in Schritt (1) applizierten Vorbehandlungsmittels auf die keratinischen Fasern für einen Zeitraum von 2 bis 45 Minuten,
(3) Ausspülen des Vorbehandlungsmittels mit Wasser,
(4) Applizieren eines Färbemittels (F) auf die keratinischen Fasern,
(5) Einwirken des in Schritt (4) applizierten Färbemittels auf die keratinischen Fasern für einen Zeitraum von 15 Sekunden bis 45 Minuten, und
(6) Ausspülen des Färbemittels mit Wasser,

wobei das Vorbehandlungsmittel in einem kosmetischen Träger
(V-1) mindestens ein Oxidationsmittel enthält, und
das Färbemittel in einem kosmetischen Träger

(F-1) mindestens ein aminofunktionalisiertes Silikonpolymer und
(F-2) mindestens ein Pigment enthält,

dadurch gekennzeichnet, dass zwischen den Schritten (3) und (4) ein Zeitraum von maximal 3 Stunden liegt

Bei den zu dieser Erfindung führenden Arbeiten hat sich gezeigt, dass besonders intensive und waschechte Farbergebnisse auf Haaren erhalten werden können, wenn die Haare durch sukzessive Anwendung der beiden Mittel (V) und (F) gefärbt wurden. Hierbei trat auch nach mehreren Haarwäschen keine unerwünschte bzw. unattraktive Nuancenverschiebung auf.

keratinisches Material

Unter keratinischem Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.

Ganz besonders bevorzugt wird unter keratinischem Material das menschliche Haar verstanden.

Mittel zur Färbung

Der Begriff "Mittel zur Färbung" wird im Rahmen dieser Erfindung für eine durch Einsatz von Pigmenten hervorgerufene Farbgebung der Keratinfasern, insbesondere des Haares, verwendet. Bei dieser Färbung lagern sich die Pigmente als farbgebende Verbindungen in einem besonders homogenen, gleichmäßigen und glatten Film an der Oberfläche der Keratinfasern ab.

Vorbehandlungsmittel (V)

Vor Anwendung des Färbemittels (F) wird im erfindungsgemäßen Verfahren das Vorbehandlungsmittel (V) auf die Keratinfasern appliziert.

Kosmetischer Träger des Vorbehandlungsmittels (V)

Das Vorbehandlungsmittel (V) ist dadurch gekennzeichnet, dass es in einem kosmetischen Träger mindestens ein Oxidationsmittel (V-1) enthält.

Als kosmetischer Träger kann für das Vorbehandlungsmittel (V) beispielsweise ein geeigneter wässriger,

alkoholischer oder wässrig-alkoholischer Träger eingesetzt werden. Zum Zwecke der Haarfärbung sind solche Träger beispielsweise Cremes, Emulsionen, Gele, Pasten oder auch tensidhaltige schäumende Lösungen, wie beispielsweise Shampoos, Schaumaerosole, Schaumformulierungen oder andere Zubereitungen, die für die Anwendung auf dem Haar geeignet sind.

Enthält das Vorbehandlungsmittel (V) das oder die Oxidationsmittel in einem wässrigen oder wasserhaltigen Träger, so besitzt das Vorbehandlungsmittel (V) bevorzugt einen mittleren Wasseranteil. Es hat sich herausgestellt, dass besonders die Vorbehandlungsmittel (V) zum Einsatz im erfindungsgemäßen Verfahren gut geeignet sind, die - bezogen auf das Gesamtgewicht des Vorbehandlungsmittel (V) - 30,0 bis 80,0 Gew.-%, bevorzugt 35,0 bis 80,0 Gew.-%, weiter bevorzugt 40,0 bis 70,0 Gew.-% und ganz besonders bevorzugt 45,0 bis 60,0 Gew.-% Wasser enthalten.

In einer Ausführungsform ist ein erfindungsgemäßes Verfahren dadurch gekennzeichnet, dass das Vorbehandlungsmittel (V) - bezogen auf das Gesamtgewicht des Vorbehandlungsmittels (V) - 30,0 bis 80,0 Gew.-%, bevorzugt 35,0 bis 80,0 Gew.-%, weiter bevorzugt 40,0 bis 70,0 Gew.-% und ganz besonders bevorzugt 45,0 bis 60,0 Gew.-% Wasser enthält.

Oxiationsmittel (V-1) bzw. (V-2) im Vorbehandlungsmittel (V)

Unter Oxidationsmitteln werden oxidierend wirkende Substanzen verstanden. Die Oxidationsmittel im Sinne der vorliegenden Erfindung sind insbesondere in der Lage, das menschliche Haar oxidativ zu verändern. Die Oxidationsmittel im Sinne der Erfindung sind von Luftsauerstoff verschieden und besitzen ein solches Oxidationspotenzial, das es ermöglicht, Disulfidbrücken innerhalb oder zwischen den Proteinen des Haarkeratins zu knüpfen und das natürliche Farbpigment Melanin oxidativ aufzuhellen.

Im Rahmen einer Ausführungsform kommt als Oxidationsmittel besonders bevorzugt Wasserstoffperoxid und/oder mindestens ein Anlagerungsprodukt davon, insbesondere an anorganische oder organische Verbindungen, wie beispielsweise Natriumperborat, Natriumpercarbonat, Magnesiumpercarbonat, Natriumpercarbamid, Polyvinylpyrrolidon·n $H_2O_2$ (n ist eine positive ganze Zahl größer 0), Harnstoffperoxid und Melaminperoxid in Frage.

Im Rahmen einer weiteren Ausführungsform können besonders bevorzugt auch Persulfate und/oder deren Salze als Oxidationsmittel im Vorbehandlungsmittel (V) eingesetzt werden. Ganz besonders bevorzugt ist der Einsatz eines oder mehrerer Persulfate aus der Gruppe aus Ammoniumperoxodisulfat, Kaliumperoxodisulfat und Natriumperoxodisulfat.

Unter Ammoniumperoxodisulfat, das alternativ auch als Ammoniumpersulfat bezeichnet werden kann, wird das Persufat mit der Summenfomel $(NH_4)_2S_2O_8$ verstanden.

Unter Kaliumperoxodisulfat, das alternativ auch als Kaliumperuslfat bezeichnet werden kann, wird das Persulfat mit der Summenformel $K_2S_2O_8$ verstanden.

Unter Natriumperoxodisulfat, das alternativ auch als Natriumpersulfat bezeichnet werden kann, wird das Persulfat mit der Summenformel $Na_2S_2O_8$ verstanden.

Im Rahmen einer ganz besonders bevorzugten Ausführungsform ist ein erfindungsgemäßes Verfahren dadurch gekennzeichnet, dass das Vorbehandlungmittel (V) mindestens ein Oxidationsmittel (V-1) aus der Gruppe aus Wasserstoffperoxid, Ammoniumperoxodisulfat, Kaliumperoxodisulfat und Natriumperoxodisulfat enthält.

Bei der Durchführung der zu dieser Erfindung führenden Versuche hat sich herausgestellt, das insbesondere dann eine starke und effektive Verbesserung der Waschechtheit erzielt werden konnte, wenn im erfindungsgemäßen Verfahren ein Vorbehandlungsmittel (V) eingesetzt wurde, welches eine Kombination verschiedener Oxidationsmittel enthielt. Besonders gute Ergebnisse wurden bei Einsatz eines Vorbehandlungsmittels (V) enthaltend die Kombination aus Wasserstoffperoxid (V-1) und mindestens ein Persulfat (V-2) aus der Gruppe aus Ammoniumperoxodisulfat, Kaliumperoxodisulfat und Natriumperoxodisulfat erhalten.

Im Rahmen einer weiteren ganz besonders bevorzugten Ausführungsform ist ein erfindungsgemäßes Verfahren dadurch gekennzeichnet, dass das Vorbehandlungsmittel (V) enthält

(V-1) Wasserstoffperoxid und
(V-2) mindestens ein Persulfat aus der Gruppe aus Ammoniumperoxodisulfat, Kaliumperoxodisulfat und Natriumperoxodisulfat.

Im Rahmen einer weiteren ganz besonders bevorzugten Ausführungsform ist ein erfindungsgemäßes Verfahren dadurch gekennzeichnet, dass das Vorbehandlungsmittel (V) enthält

(V-1) Wasserstoffperoxid und
(V-2) Ammoniumperoxodisulfat.

Im Rahmen einer weiteren ganz besonders bevorzugten Ausführungsform ist ein erfindungsgemäßes Verfahren

dadurch gekennzeichnet, dass das Vorbehandlungsmittel (V) enthält

(V-1) Wasserstoffperoxid und
(V-2) Kaliumperoxodisulfat.

Im Rahmen einer weiteren ganz besonders bevorzugten Ausführungsform ist ein erfindungsgemäßes Verfahren dadurch gekennzeichnet, dass das Vorbehandlungsmittel (V) enthält

(V-1) Wasserstoffperoxid und
(V-2) Natriumperoxodisulfat.

Im Rahmen einer weiteren ganz besonders bevorzugten Ausführungsform ist ein erfindungsgemäßes Verfahren dadurch gekennzeichnet, dass das Vorbehandlungsmittel (V) enthält

(V-1) Wasserstoffperoxid und
(V-2) Ammoniumperoxodisulfat und Kaliumperoxodisulfat.

Im Rahmen einer weiteren ganz besonders bevorzugten Ausführungsform ist ein erfindungsgemäßes Verfahren dadurch gekennzeichnet, dass das Vorbehandlungsmittel (V) enthält

(V-1) Wasserstoffperoxid und
(V-2) Ammoniumperoxodisulfat und Natriumperoxodisulfat.

Im Rahmen einer weiteren ganz besonders bevorzugten Ausführungsform ist ein erfindungsgemäßes Verfahren dadurch gekennzeichnet, dass das Vorbehandlungsmittel (V) enthält

(V-1) Wasserstoffperoxid und
(V-2) Kaliumperoxodisulfat und Natriumperoxodisulfat.

Im Rahmen einer weiteren ganz besonders bevorzugten Ausführungsform ist ein erfindungsgemäßes Verfahren dadurch gekennzeichnet, dass das Vorbehandlungsmittel (V) enthält

(V-1) Wasserstoffperoxid und
(V-2) Ammoniumperoxodisulfat und Kaliumperoxodisulfat und Natriumperoxodisulfat.

Erfindungsgemäß kann das oxidative kosmetische Vorbehandlungsmittel zusätzlich auch noch mindestens einen Katalysator als optionalen Bestandteil enthalten, der die Oxidation des Substrats, wie beispielsweise das im Keratinmaterial befindliche Melanin, aktiviert. Solche Katalysatoren sind z.B. Metallionen, Iodide, Chinone oder bestimmte Enzyme.

Geeignete Metallionen sind beispielsweise $Zn^{2+}$, $Cu^{2+}$, $Fe^{2+}$, $Fe^{3+}$, $Mn^{2+}$, $Mn^{4+}$, $Li^{+}$, $Mg^{2+}$, $Ca^{2+}$ und $Al^{3+}$. Besonders geeignet sind dabei $Zn^{2+}$, $Cu^{2+}$ und $Mn^{2+}$. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation von Farbstoffvorprodukten sind die sogenannten 2-Elektronen-Oxidoreduktasen in Kombination mit den dafür spezifischen Substraten, z.B.

- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,

- Aminosäure-Oxidase und Aminosäuren.

Durch Wahl der geeigneten Mengenbereiche an Oxidationsmittel(n) (V-1) (bzw. (V-1) und (V-2)) im Vorbehandlungsmittel (V) kann die Größe des Einflusses, den das Vorbehandlungsmittel (V) auf die Waschechtheit des nachfolgend angewendeten Färbemittels (F) besitzt, gezielt gesteuert werden. In diesem Zusammenhang hat sich herausgestellt, dass die Waschechtheit umso besser ist, je höher die Einsatzmenge an Oxidationsmittel(n) im Vorbehandlungsmittel (V) ist. Andererseits steigt jedoch auch die Schädigung des Keratinmaterials bzw. der Keratinfasern/-Haare mit Erhöhung der Einsatzmenge an Oxidationsmitteln. Um zwischen diesen beiden Effekten die optimale Balance zu finden, hat es sich als ganz besonders bevorzugt herausgestellt, das bzw. die Oxidationsmittel in ganz bestimmten Mengenbereichen im Vorbehandlungsmittel einzusetzen.

Bevorzugt enthält das erfindungsgemäße Vorbehandlungsmittel - bezogen auf das Gesamtgewicht des Vorbehandlungsmittels - 0,1 bis 12,0 Gew.-%, weiter bevorzugt 0,5 bis 10,0 Gew.-%, noch weiter bevorzugt 1,5 bis 8,0 Gew.-% und ganz besonders bevorzugt 3,0 bis 6,0 Gew.-% Wasserstoffperoxid (V-1).

Im Rahmen einer weiteren ganz besonders bevorzugten Ausführungsform ist ein erfindungsgemäßes Verfahren dadurch gekennzeichnet, dass das Vorbehandlungsmittel (V) - bezogen auf das Gesamtgewicht des Vorbehandlungsmittels - (V-1) 0,1 bis 12,0 Gew.-% bevorzugt 0,5 bis 10,0 Gew.-%, weiter bevorzugt 1,5 bis 8,0 Gew.-% und ganz besonders bevorzugt 3,0 bis 6,0 Gew.-% Wasserstoffperoxid enthält.

Bevorzugt enthält das erfindungsgemäße Vorbehandlungsmittel (V) - bezogen auf das Gesamtgewicht des Vorbehandlungsmittels - ein oder mehrere Persulfate (V-2) aus der Gruppe aus Ammoniumperoxodisulfat, Kaliumperoxodisulfat und Natriumperoxodisulfat in einer Gesamtmenge von 5,0 bis 30,0 Gew.-%, weiter bevorzugt von 8,0 bis 27,0 Gew.-%, noch weiter bevorzugt von 11,0 bis 24,0 Gew.-% und ganz besonders von 14,0 bis 21,0 Gew.-%.

Im Rahmen einer weiteren ganz besonders bevorzugten Ausführungsform ist ein erfindungsgemäßes Verfahren dadurch gekennzeichnet, dass das Vorbehandlungsmittel (V) - bezogen auf das Gesamtgewicht des Vorbehandlungsmittels - (V2) ein oder mehrere Persulfate aus der Gruppe aus Ammoniumperoxodisulfat, Kaliumperoxodisulfat und Natriumperoxodisulfat in einer Gesamtmenge von 5,0 bis 30,0 Gew.-%, bevorzugt von 8,0 bis 27,0 Gew.-%, weiter bevorzugt von 11,0 bis 24,0 Gew.-% und ganz besonders von 14,0 bis 21,0 Gew.-% enthält.

Färbemittel (F)

Im Anschluss an die Anwendung des Vorbehandlungsmittels (V) wird im erfindungsgemäßen Verfahren nun das Färbemittel (F) auf die Keratinfasern appliziert.

Kosmetischer Träger des Färbemittels (F)

Das Färbemittel (F) enthält mindestens ein aminofunktionalisiertes Silikonpolymer (F-1) und mindestens ein Pigment (F-2) in einem kosmetischen Träger.

Als kosmetischer Träger kann für das Färbemittel (F) beispielsweise ein geeigneter wässriger, alkoholischer oder wässrig-alkoholischer Träger eingesetzt werden. Zum Zwecke der Haarfärbung sind solche Träger beispielsweise Cremes, Emulsionen, Gele, Pasten oder auch tensidhaltige schäumende Lösungen, wie beispielsweise Shampoos, Schaumaerosole, Schaumformulierungen oder andere Zubereitungen, die für die Anwendung auf dem Haar geeignet sind.

Enthält das Färbemittel (F) das bzw. die aminofunktionalisierte Silikonpolymere (F-1) und das bzw. die Pigmente (F-2) in einem wässrigen oder wasserhaltigen Träger, so besitzt das Färbemittel (F) bevorzugt einen hohen Wasseranteil. Es hat sich herausgestellt, dass besonders die Färbemittel (F) zum Einsatz im erfindungsgemäßen Verfahren gut geeignet sind, die - bezogen auf das Gesamtgewicht des Vorbehandlungsmittel (V) - 50,0 bis 99,0 Gew.-%, bevorzugt 60,0 bis 99,0 Gew.-%, weiter bevorzugt 70,0 bis 99,0 Gew.-% und ganz besonders bevorzugt 80,0 bis 99,0 Gew.-% Wasser enthalten.

In einer Ausführungsform ist ein erfindungsgemäßes Verfahren dadurch gekennzeichnet, dass das Färbemittel (F) - bezogen auf das Gesamtgewicht des Färbemittels (F) - 50,0 bis 99,0 Gew.-%, bevorzugt 60,0 bis 99,0 Gew.-%, weiter bevorzugt 70,0 bis 99,0 Gew.-% und ganz besonders bevorzugt 80,0 bis 99,0 Gew.-% Wasser enthält.

aminofunktionalisiertes Silikonpolymer (F-1) im Färbemittel (F)

Als ersten erfindungswesentlichen Inhaltsstoff (F-1) enthält das Färbemittel (F) mindestens ein aminofunktionalisiertes Silikonpolymer. Das aminofunktionalisiertes Silikonpolymer kann alternativ auch als Aminosilikon oder Amodimethicone bezeichnet werden.

Silikonpolymere sind im allgemeinen Makromoleküle mit einem Molekulargewicht von mindestens 500 g/mol, bevorzugt mindestens 1000 g/mol, weiter bevorzugt von mindestens 2500 g/mol, besonders bevorzugt von mindestens

5000 g/mol, welche sich wiederholende organische Einheiten umfassen.

Das maximale Molekulargewicht des Silikonpolymers hängt von dem Polymerisationsgrad (Anzahl der polymerisierten Monomere) und der Ansatzgröße ab und wird durch die Polymerisationsmethode mitbestimmt. Im Sinne der vorliegenden Erfindung ist es bevorzugt, wenn das maximale Molekulargewicht des Silikonpolymers nicht mehr als $10^7$ g/mol, bevorzugt nicht mehr als $10^6$ g/mol und besonders bevorzugt nicht mehr als $10^5$ g/mol beträgt.

Die Silikonpolymere umfassen viele Si-O-Wiederholungseinheiten, wobei die Si-Atome organische Reste wie beispielsweise Alkylgruppen oder substituierte Alkylgruppen tragen können. Alternativ wird ein Silikonpolymer daher auch als Polydimethylsiloxan bzw. als Derivat hiervon bezeichnet.

In Entsprechung des hohen Molekulargewichts der Silikonpolymere basieren diese auf mehr als 10 Si-O Wiederholungseinheiten, bevorzugt mehr als 50 Si-O-Wiederholungseinheiten und besonders bevorzugt mehr als 100 Si-O-Wiederholungseinheiten, ganz besonders bevorzugt mehr als 500 Si-O-Wiederholungseinheiten.

Unter einem aminofunktionalisierten Silikonpolymer wird ein funktionalisiertes Silikon verstanden, welches mindestens eine Struktureinheit mit einer Aminogruppe trägt. Bevorzugt trägt das aminofunktionalisierte Silikonpolymer mehrere Struktureinheiten mit jeweils mindestens einer Aminogruppe. Unter einer Aminogruppe wird eine primäre Aminogruppe, eine sekundäre Aminogruppe und eine tertiäre Aminogruppe verstanden. Alle diese Aminogruppen können im sauren Milieu protoniert werden und liegen dann in ihrer kationischen Form vor.

Prinzipiell konnten gute Effekte mit aminofunktionalisierten Silikonpolymeren (F-1) erzielt werden, wenn diese mindestens eine primäre, mindestens eine sekundäre und/oder mindestens eine tertiäre Aminogruppe tragen. Färbungen mit der besten Waschechtheit wurden jedoch beobachtet, wenn ein aminofunktionalisiertes Silikonpolymer (F-1) im Färbemittel (F) eingesetzt wurde, welches mindestens eine sekundäre Aminogruppe enthält.

In einer ganz besonders bevorzugten Ausführungsform ist ein erfindungsgemäßes Verfahren dadurch gekennzeichnet, dass das Färbemittel (F) mindestens ein aminofunktionalisiertes Silikonpolymer (F-1) mit mindestens eine sekundären Aminogruppe enthält.

Die sekundäre Aminogruppe(n) kann bzw. können sich an verschiedenen Positionen des aminofunktionalisierten Silikonpolymers befinden. Ganz besonders gute Effekte wurden gefunden, wenn ein aminofunktionalisiertes Silikonpolymer (F-1) eingesetzt wurde, dass mindestens eine, bevorzugt mehrere Struktureinheiten der Formel (Si-Amino) besitzt.

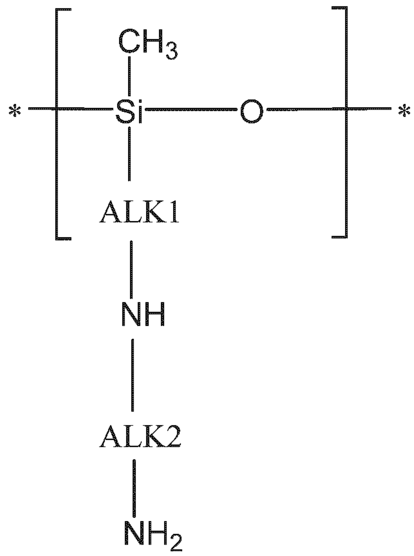

(Si-Amino)

In den Struktureinheiten der Formel (Si-Amino) steht die Kürzel ALK1 und ALK2 unabhängig voneinander für eine lineare oder verzweigte, zweiwertige $C_1$-$C_{20}$-Alkylengruppe.

In einer weiteren ganz besonders bevorzugten Ausführungsform ist ein erfindungsgemäßes Verfahren dadurch gekennzeichnet, dass das Färbemittel (F) mindestens ein aminofunktionalisiertes Silikonpolymer (F-1) enthält, das mindestens eine Struktureinheit der Formel (Si-Amino) umfasst,

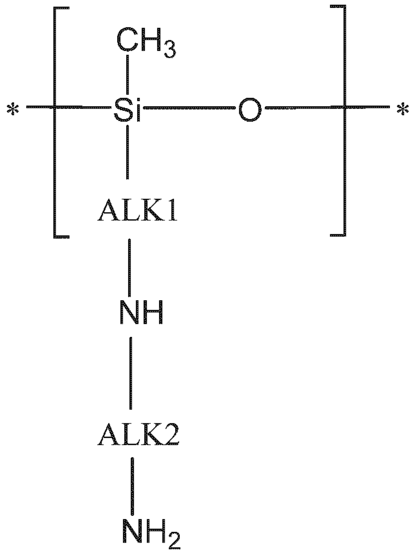

(Si-Amino)

wobei

ALK1 und ALK2 unabhängig voneinander für eine lineare oder verzweigte, zweiwertige $C_1$-$C_{20}$-Alkylengruppe stehen.

Die mit einem Stern (*) gekennzeichneten Position geben hierbei jeweils die Bindung zu weiteren Struktureinheiten des Silikonpolymers an. Beispielsweise kann das dem Stern benachbarte Silicium-Atom an ein weiteres Sauerstoffatom gebunden sein, und das dem Stern benachbarte Sauerstoffatom kann an ein weiteres Siliciumatom oder auch an eine $C_1$-$C_6$-Alkylgruppe gebunden sein.

Eine zweiwertige $C_1$-$C_{20}$-Alkylengruppe kann alternativ auch als eine divalente oder zweibindige $C_1$-$C_{20}$-Alkylengruppe bezeichnet werden, womit gemeint ist, dass jede Gruppierung ALK1 bzw. AK2 zwei Bindungen eingehen kann.

Im Fall von ALK1 erfolgt eine Bindung vom Silicium-Atom zur Gruppierung ALK1, und die zweite Bindung besteht zwischen ALK1 und der sekundären Aminogruppe.

Im Fall von ALK2 erfolgt eine Bindung von der sekundären Aminogruppe zur Gruppierung ALK2, und die zweite Bindung besteht zwischen ALK2 und der primären Aminogruppe.

Beispiele für eine lineare zweiwertige $C_1$-$C_{20}$-Alkylengruppe sind beispielsweise die Methylen-gruppe ($-CH_2-$), die Ethylengruppe ($-CH_2-CH_2-$), die Propylengruppe ($-CH_2-CH_2-CH_2-$) und die Butylengruppe ($-CH_2-CH_2-CH_2-CH_2-$). Die Propylengruppe ($-CH_2-CH_2-CH_2-$) ist besonders bevorzugt. Ab einer Kettenlänge von 3 C-Atomen können zweiwertige Alkylengruppen auch verzweigt sein. Beispiele für verzweigte, zweiwertige $C_3$-$C_{20}$-Alkylengruppen sind ($-CH_2-CH(CH_3)-$) und ($-CH_2-CH(CH_3)-CH_2-$).

In einer weiteren besonders bevorzugten Ausführungsform stellen die Struktureinheiten der Formel (Si-Amino) Wiederholungseinheiten im aminofunktionalisierten Silikonpolymer (F-1) dar, so dass das Silikonpolymer mehrer Struktureinheiten der Formel (Si-Amino) umfasst.

Im Folgenden werden besonders gut geeignete aminofunktionalisierte Silikonpolymere (F-1) mit mindestens einer sekundären Aminogruppe aufgelistet.

Färbungen mit den allerbesten Waschechtheiten konnten erhalten werden, wenn im erfindungsgemäßen Verfahren mindestens ein Färbemittel (F) auf dem keratinischen Material appliziert wurde, das mindestens ein aminofunktionalisiertes Silikonpolymer (F-1) enthält, das Struktureinheiten der Formel (Si-I) und der Formel (Si-II) umfasst

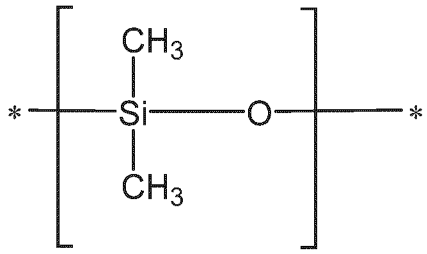

(Si-I)

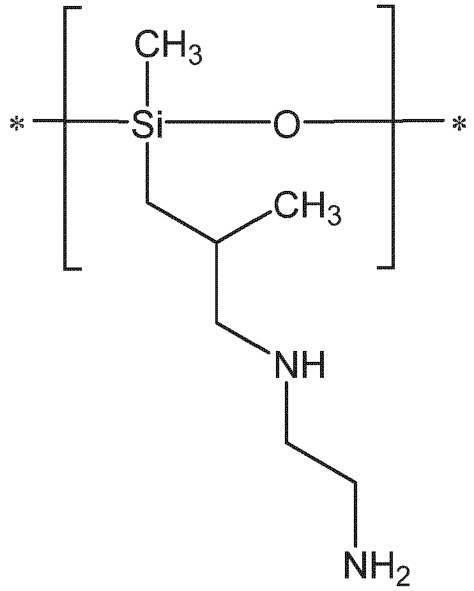

(Si-II).

In einer weiteren explizit ganz besonders bevorzugten Ausführungsform ist ein erfindungsgemäßes Verfahren dadurch gekennzeichnet, dass das Färbemittel (F) mindestens ein aminofunktionalisiertes Silikonpolymer (F-1) enthält, das Struktureinheiten der Formel (Si-I) und der Formel (Si-II) umfasst

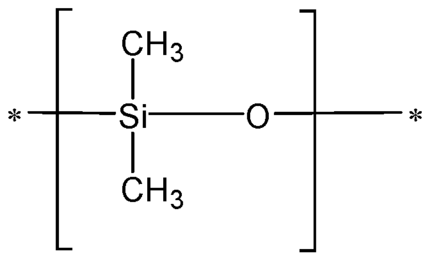

(Si-I)

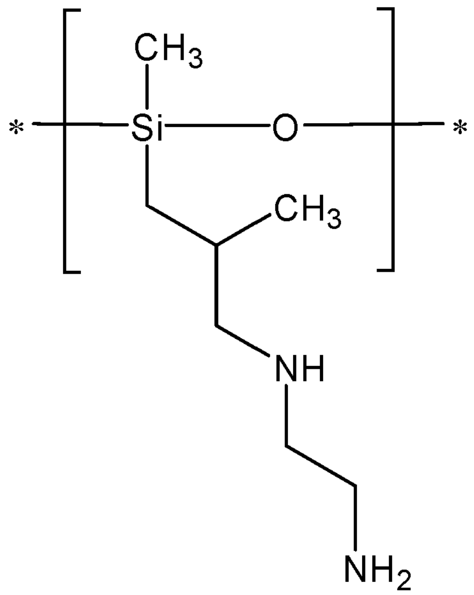

(Si-II).

Ein entsprechendes aminofunkionalisiertes Silikonpolymer mit den Struktureinheiten (Si-I) und (Si-II) ist beispielweise das Handelsprodukt DC 2-8566 bzw. Dowsil 2-8566 Amino Fluid, das von der Firma Dow Chemical Company komerziell vertrieben wird und welches die Benennung "Siloxanes and Silicones, 3-[(2-Aminoethyl)amino]-2-methylpropyl Me, Di-Me-Siloxane" sowie die CAS-Nummer 106842-44-8 trägt. in weiteres besonders bevorzugtes Handelsprodukt ist Dowsil AP-8658 Amino Fluid, das ebenfalls von der Firma Dow Chemical Company kommerziell vertrieben wird.

Im Rahmen einer weiteren bevorzugten Ausführungsform ist ein erfindungsgemäßes Verfahren gekennzeichnet durch die Anwendung eines Färbemittels (F) auf dem keratinischem Material, wobei das Färbemittel (F) mindestens ein aminofunktionelles Silikonpolymer (F-1) der Formel der Formel (Si-III) enthält,

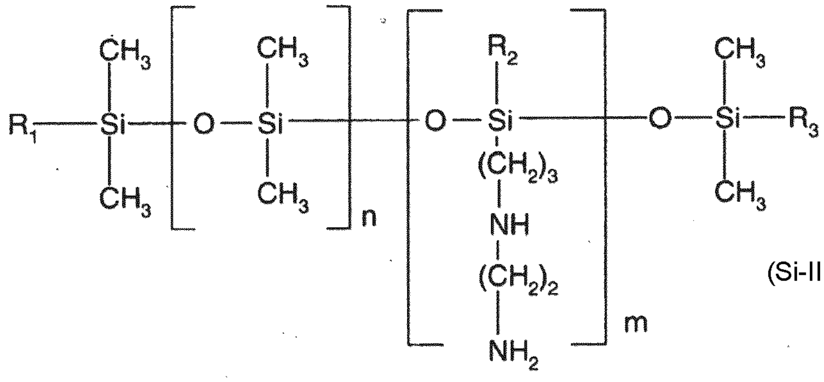

(Si-III)

wobei

- m und n bedeuten Zahlen, die so gewählt sind, daß die Summe (n + m) im Bereich von 1 bis 1000 liegt,
- n ist eine Zahl im Bereich von 0 bis 999 und m ist eine Zahl im Bereich von 1 bis 1000,
- R1, R2 und R3, die gleich oder verschieden sind, bedeuten eine Hydroxygruppe oder eine C1-4-Alkoxygruppe,
- wobei mindestens eine der Gruppen R1 bis R3 eine Hydroxygruppe bedeutet;

Weitere erfindungsgemäß bevorzugte Verfahren sind gekennzeichnet durch die Anwendung eines Färbemittels (F) auf dem keratinischem Material, wobei das Färbemittel (F) mindestens aminofunktionelles Silikonpolymer (F-1) der Formel der Formel (Si-IV) enthält,

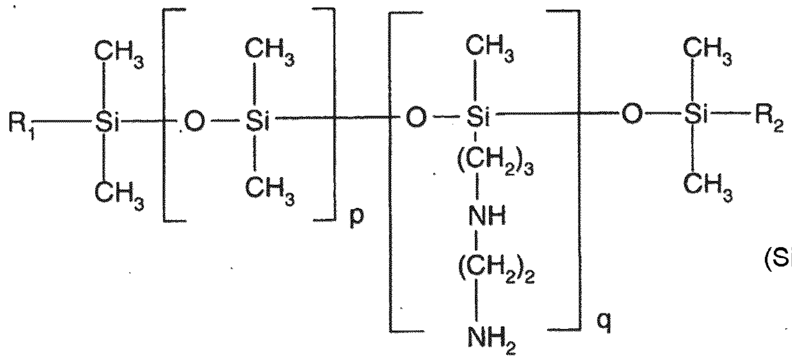

(Si-IV)

in der

- p und q bedeuten Zahlen, die so gewählt sind, daß die Summe (p + q) im Bereich von 1 bis 1000 liegt,
- p ist eine Zahl im Bereich von 0 bis 999 und q ist eine Zahl im Bereich von 1 bis 1000,
- R1 und R2, die verschieden sind, bedeuten eine Hydroxygruppe oder eine C1-4-Alkoxygruppe, wobei mindestens eine der Gruppen R1 bis R2 eine Hydroxygruppe bedeutet.

Die Silikone der Formeln (Si-III) und (Si-IV) unterscheiden sich durch die Gruppierung am Si-Atom, das die stickstoffhaltige Gruppe trägt: In Formel (Si-III) bedeutet R2 eine Hydroxygruppe oder eine C1-4-Alkoxygruppe, während der Rest in Formel (Si-IV) eine Methylgruppe ist. Die einzelnen Si-Gruppierungen, die mit den Indices m und n bzw. p und q gekennzeichnet sind, müssen nicht als Blöcke vorliegen, vielmehr können die einzelnen Einheiten auch statistisch verteilt vorliegen, d.h. in den Formeln (Si-III) und (Si-IV) ist nicht zwingend jedes R1-$Si(CH_3)_2$-Gruppe an eine -[O-$Si(CH_3)_2$]-Gruppierung gebunden.

Als besonders wirkungsvoll im Hinblick auf die gewünschten Effekte haben sich auch erfindungsgemäßen Verfahren erwiesen, in welchen ein Färbemittel (F) auf den Keratinfasern appliziert wird, welches mindestens ein aminofunktionelles Silikonpolymer (F-1) der Formel der Formel (Si-V) enthält

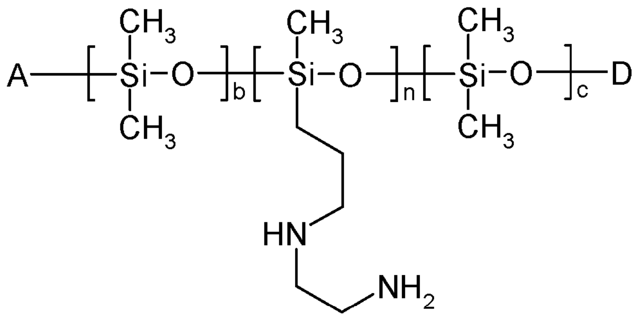

(Si-V),

in der

A für eine Gruppe -OH, $-O-Si(CH_3)_3$,$-O-Si(CH_3)_2OH$ ,$-O-Si(CH_3)_2OCH_3$ steht,
D für eine Gruppe -H, $-Si(CH_3)_3$,$-Si(CH_3)_2OH$, $-Si(CH_3)_2OCH_3$ steht,
b, n und c für ganze Zahlen zwischen 0 und 1000 stehen,
mit den Maßgaben

- $n > 0$ und $b + c > 0$
- mindestens eine der Bedingungen A = -OH bzw. D = -H ist erfüllt.

In der vorstehend genannten Formel (Si-V) sind die einzelnen Siloxaneinheiten mit den Indices b, c und n statistisch verteilt, d.h. es muß sich nicht zwingend um Blockcopolymere handeln.

Das Färbemittel (F) kann weiterhin auch ein oder mehrere verschiedene aminofunktionalisierte Silikonpolymere enthalten, die durch die Formel (Si-VI)

$$M(R_aQ_bSiO_{(4-a-b)/2})\times(R_cSiO_{(4-c)/2)y}M \qquad \text{(Si-VI)}$$

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel $-R^1HZ$ ist, worin $R^1$ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe ist, wie sie im Stand der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl;

Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von $R^1$ schließen Methylen, Ethylen, Propylen, Hexamethylen, Decamethylen, - $CH_2CH(CH_3)CH_2$-, Phenylen, Naphthylen, $-CH_2CH_2SCH_2CH_2-$, $-CH_2CH_2OCH_2-$, $-OCH_2CH_2-$, $-OCH_2CH_2CH_2-$, $-CH_2CH(CH_3)C(O)OCH_2-$, $-(CH_2)_3CC(O)OCH_2CH_2-$, $-C_6H_4C_6H_4-$, $-C_6H_4CH_2C_6H_4-$; und $-(CH_2)_3C(O)SCH_2CH_2-$ ein.

Z ist ein organischer, aminofunktioneller Rest, enthaltend mindestens eine funktionelle Aminogruppe. Eine mögliche Formel für Z ist $NH(CH_2)_zNH_2$, worin z 1 oder mehr ist. Eine andere mögliche Formel für Z ist $-NH(CH_2)_z(CH_2)_{zz}NH$, worin sowohl z als auch zz unabhängig 1 oder mehr sind, wobei diese Struktur Diamino-Ringstrukturen umfaßt, wie Piperazinyl. Z ist am bevorzugtesten ein $-NHCH_2CH_2NH_2$-Rest. Eine andere mögliche Formel für Z ist - $N(CH_2)_z(CH_2)_{zz}NX_2$ oder $-NX_2$, worin jedes X von $X_2$ unabhängig ausgewählt ist aus der Gruppe bestehend aus Wasserstoff und Alkylgruppen mit 1 bis 12 Kohlenstoffatomen, und zz 0 ist.

Q ist am bevorzugtesten ein polarer, aminfunktioneller Rest der Formel $-CH_2CH_2CH_2NHCH_2CH_2NH_2$. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der $R_aQ_b SiO_{(4-}$

$_{a-b)/2}$-Einheiten zu den $R_cSiO_{(4-c)/2}$-Einheiten liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.

Im Rahmen einer besonders bevorzugten Ausführungsform ist ein erfindungsgemäßes Verfahren gekennzeichnet durch die Anwendung eines Färbemittels (F) auf dem keratinischem Material, wobei das Färbemittel (F) ein aminofunktionelles Silikonpolymer der Formel (Si-VII)

$$R'_aG_{3-a}\text{-}Si(OSiG_2)_n\text{-}(OSiG_bR'_{2-b})_m\text{-}O\text{-}SiG_{3-a}\text{-}R'_a \qquad \text{(Si-VII)},$$

enthält, worin bedeutet:

- G ist-H, eine Phenylgruppe, -OH, $-O-CH_3$, $-CH_3$, $-O-CH_2CH_3$, $-CH_2CH_3$, $-O-CH_2CH_2CH_3$,$-CH_2CH_2CH_3$, $-O-CH(CH_3)_2$, $-CH(CH_3)_2$, $-O-CH_2CH_2CH_2CH_3$, $-CH_2CH_2CH_2CH_3$, $-O-CH_2CH(CH_3)_2$, $-CH_2CH(CH_3)_2$, $-O-CH(CH_3)CH_2CH_3$, $-CH(CH_3)CH_2CH_3$, $-O-C(CH_3)_3$, $-C(CH_3)_3$ ;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus

  - $-Q-N(R'')-CH_2-CH_2-N(R'')_2$
  - $-Q-N(R'')_2$
  - $-Q-N^+(R'')_3A^-$
  - $-Q-N^+H(R'')_2\,A^-$
  - $-Q-N^+H_2(R'')A^-$
  - $-Q-N(R'')-CH_2-CH_2-N^+R''H_2A^-$,

  wobei jedes Q für eine chemische Bindung, $-CH_2-$, $-CH_2-CH_2-$, $-CH_2CH_2CH_2-$, $-C(CH_3)_2-$, $-CH_2CH_2CH_2CH_2-$, $-CH_2C(CH_3)_2-$, $-CH(CH_3)CH_2CH_2-$ steht,
  R'' für gleiche oder verschiedene Reste aus der Gruppe -H, -Phenyl, -Benzyl, $-CH_2-CH(CH_3)Ph$, der $C_{1-20}$-Alkylreste, vorzugsweise $-CH_3$, $-CH_2CH_3$, $-CH_2CH_2CH_3$, $-CH(CH_3)_2$, $-CH_2CH_2CH_2H_3$, $-CH_2CH(CH_3)_2$, $-CH(CH_3)CH_2CH_3$, $-C(CH_3)_3$, steht und A ein Anion repräsentiert, welches vorzugsweise ausgewählt ist aus Chlorid, Bromid, Iodid oder Methosulfat.

Im Rahmen einer weiteren bevorzugten Ausführungsform ist ein erfindungsgemäßes Verfahren gekennzeichnet durch die Anwendung eines Färbemittels (F) auf dem keratinischem Material, wobei das Färbemittel (F) mindestens ein aminofunktionelles Silikonpolymer (F-1) der Formel (Si-VIIa) enthält,

$$(CH_3)_3Si\text{-}[O\text{-}Si(CH_3)_2]_n[OSi(CH_3)]_m\text{-}OSi(CH_3)_3 \qquad \text{(Si-VIIa)},$$

$$|$$

$$CH_2CH(CH_3)CH_2NH(CH_2)_2NH_2$$

worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Trimethylsilylamodimethicone bezeichnet.

Im Rahmen einer weiteren bevorzugten Ausführungsform ist ein erfindungsgemäßes Verfahren gekennzeichnet durch die Anwendung eines Färbemittels (F) auf dem keratinischem Material, wobei das Färbemittel (F) mindestens ein aminofunktionelles Silikonpolymer der Formel (Si-VIIb) enthält

$$R-[Si(CH_3)_2-O]_{n1}[Si(R)-O]_m-[Si(CH_3)_2]_{n2}-R \qquad (Si\text{-}VIIb),$$

$$|$$

$$(CH_2)_3NH(CH_2)_2NH_2$$

enthalten, worin R für -OH, $-O-CH_3$ oder eine $-CH_3$-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese aminofunktionalisierten Siliconpolymere werden nach der INCI-Deklaration als Amodimethicone bezeichnet.

Unabhängig davon, welche aminofunktionellen Silicone eingesetzt werden, sind erfindungsgemäße Färbemittel (F) bevorzugt, die ein aminofunktionelles Silikonpolymer enthalten, dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silicons. Sie kann durch Titration ermittelt und auch in der Einheit mg KOH/g angegeben werden.

Weiterhin sind auch Färbemittel (F) zum Einsatz im erfindungsgemäßen Verfahren geeignet, welche ein spezielles 4-Morpholinomethyl-substituiertes Silikonpolymer (F-1) enthielten. Dieses aminofunktionalisierte Silikonpolymer umfasst Struktureinheiten der Formeln (SI-VIII) und der Formel (Si-IX)

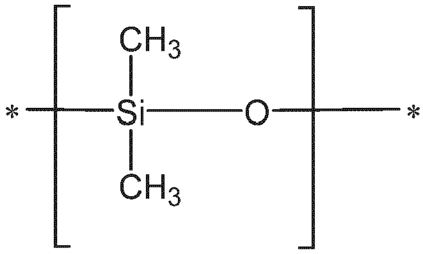

(Si-VIII)

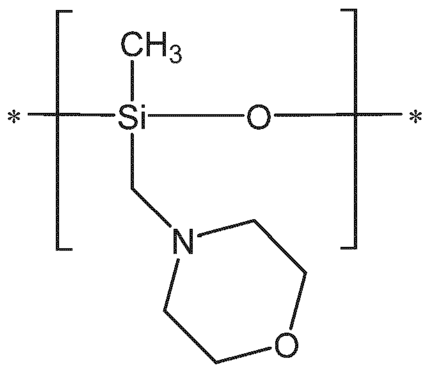

(Si-IX).

Entsprechende 4-Morpholinomethyl-substituiertes Silikonpolymere werden im folgenden beschrieben.

Ein entsprechendes aminofunktionaliserte Silikonpolymer ist unter dem Namen Amodimethicone/Morpholinomethyl Silsesquioxane Copolymer bekannt und in Form des Rohstoffes Belsil ADM 8301 E von Wacker kommerziell erhältlich.

Als 4-morpholinomethyl-substituiertes Silikon kann beispielsweise ein Silikon eingesetzt werden, welches Struktureinheiten der Formeln (Si-VIII), (Si-IX) und (Si-X) aufweist

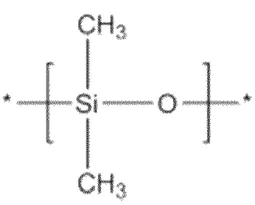

(Si-VIII),

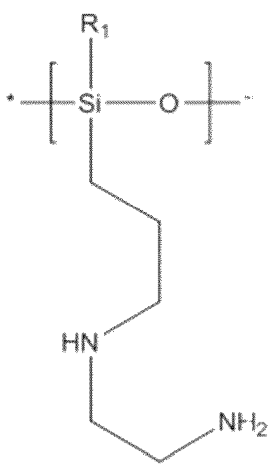

(Si-X)

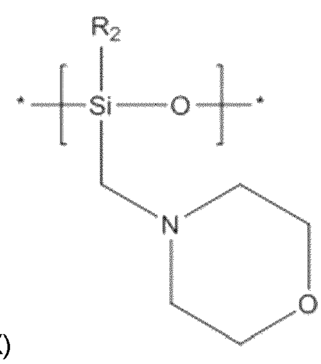

(Si-IX)

in denen

R1 für $-CH_3$, -OH, $-OCH_3$, $-O-CH_2CH_3$, $-O-CH_2CH_2CH_3$, oder $-O-CH(CH_3)_2$ steht;
R2 für $-CH_3$, -OH, oder $-OCH_3$ steht.

Besonders bevorzugte erfindungsgemäße Färbemittel (F) enthalten mindestens ein 4-morpholinomethyl-substituierten Silikons der Formel (Si-XI)

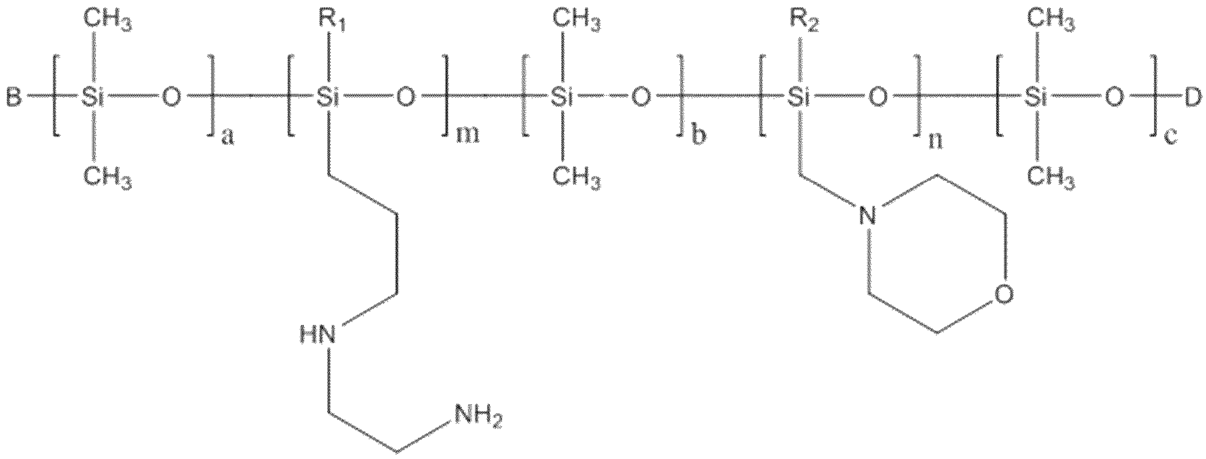

(Si-XI)

in der

R1 für $-CH_3$, -OH, $-OCH_3$, $-O-CH_2CH_3$, $-O-CH_2CH_2CH_3$, oder $-O-CH(CH_3)_2$ steht;
R2 für $-CH_3$, -OH, oder $-OCH_3$ steht.
B für eine Gruppe -OH, $-O-Si(CH_3)_3$,$-O-Si(CH_3)_2OH$ ,$-O-Si(CH_3)_2OCH_3$ steht,
D für eine Gruppe -H, $-Si(CH_3)_3$,$-Si(CH_3)_2OH$, $-Si(CH_3)_2OCH_3$ steht,

a, b und c unabhängig voneinander für ganze Zahlen zwischen 0 und 1000 stehen, mit der Maßgabe a + b + c> 0
m und n unabhängig voneinander für ganze, Zahlen zwischen 1 und 1000 stehen mit den Maßgabe, daß

- mindestens eine der Bedingungen B = -OH bzw. D = -H erfüllt ist,
- die Einheiten a, b, c, m und n statistisch oder blockweise im Molekül verteilt vorliegen.

Strukturformel (Si-XI) soll verdeutlichen, daß die Siloxangruppen n und m nicht zwingend direkt an eine Endgruppierung B bzw. D gebunden sein müssen. Vielmehr gilt in bevorzugten Formeln (Si-VI) a > 0 oder b > 0 und in besonders bevorzugten Formeln (Si-VI) a > 0 und c > 0, d.h. die terminale Gruppierung B bzw. D ist vorzugsweise an eine Dimethylsiloxy-Gruppierung gebunden. Auch in Formel (Si-VI) sind die Siloxaneinheiten a, b, c, m und n vorzugsweise statistisch verteilt.

Die durch Formel (Si-VI) dargestellten erfindungsgemäß eingesetzten Silikone können trimethylsilylterminiert sein (D oder B = $-Si(CH_3)_3$), sie können aber auch zweiseitig dimethylsilylhydroxy- oder einseitig dimethylsilylhydroxy- und dimethylsilylmethoxy-terminiert sein. Im Rahmen der vorliegenden Erfindung besonders bevorzugt eingesetzte Silikone sind ausgewählt aus Silikonen, in denen

| | |
|---|---|
| B = $-O-Si(CH_3)_2OH$ und | D = $-Si(CH_3)_3$ |
| B = $-O-Si(CH_3)_2OH$ und | D = $-Si(CH_3)_2OH$ |
| B = $-O-Si(CH_3)_2OH$ und | D = $-Si(CH_3)_2OCH_3$ |
| B = $-O-Si(CH_3)_3$ | und D = $-Si(CH_3)_2OH$ |
| B = $-O-Si(CH_3)_2OCH_3$ | und D = $-Si(CH_3)_2OH$ |

bedeutet. Diese Silikone führen zu exorbitanten Verbesserungen der Haareigenschaften der mit den erfindungsgemäßen Mitteln behandelten Haare, und zu einem gravierend verbesserten Schutz bei oxidativer Behandlung.

Es hat sich als besonders vorteilhaft herausgestellt, wenn das erfindungsgemäße Färbemittel das oder die aminofunktionalisierten Silikonpolymere (F-1) in bestimmten Mengenbereichen enhält. Besonders gute Ergebnisse

konnten erhalten werden, wenn das Färbemittel - bezogen auf das Gesamtgewicht des Färbemittels - ein oder mehrere aminofunktionalisierte Silikonpolymere (F-1) in einer Gesamtmenge von 0,1 bis 8,0 Gew.-%, bevorzugt 0,2 bis 5,0 Gew.-%, weiter bevorzugt von 0,3 bis 3,0 Gew.-% und ganz besonders bevorzugt von 0,4 bis 2,5 Gew.-% enthält.

Im Rahmen einer weiteren besonders bevorzugten Ausführungsform ist ein erfindungsgemäßes Verfahren dadurch gekennzeichnet, dass das Färbemittel (F) - bezogen auf das Gesamtgewicht des Färbemittels - ein oder mehrere aminofunktionalisierte Silikonpolymere (F-1) in einer Gesamtmenge von 0,1 bis 8,0 Gew.-%, bevorzugt 0,2 bis 5,0 Gew.-%, weiter bevorzugt von 0,3 bis 3,0 Gew.-% und ganz besonders bevorzugt von 0,4 bis 2,5 Gew.-% enthält.

Pigmente (F-2) im Färbemittel (F)

Als zweiten erfindungswesentlichen Bestandteil enthält das im erfindungsgemäßen Verfahren eingesetzte Färbemittel (F) mindestens ein Pigment.

Unter Pigmenten im Sinne der vorliegenden Erfindung werden farbgebende Verbindungen verstanden, welche bei 25 °C in Wasser eine Löslichkeit von weniger als 0,5 g/L, bevorzugt von weniger als 0,1 g/L, noch weiter bevorzugt von weniger als 0,05 g/L besitzen. Die Wasserlöslichkeit kann beispielsweise mittels der nachfolgend beschriebenen Methode erfolgen: 0,5 g des Pigments werden in einem Becherglas abgewogen. Ein Rührfisch wird hinzugefügt. Dann wird ein Liter destilliertes Wasser hinzugegeben. Dieses Gemisch wird unter Rühren auf einem Magnetrührer für eine Stunde auf 25 °C erhitzt. Sind in der Mischung nach diesem Zeitraum noch ungelöste Bestandteile des Pigments sichtbar, so liegt die Löslichkeit des Pigments unterhalb von 0,5 g/L. Sofern sich die Pigment-Wasser-Mischung aufgrund der hohen Intensität des gegebenenfalls feindispergiert vorliegenden Pigments nicht visuell beurteilten lässt, wird die Mischung filtriert. Bleibt auf dem Filterpapier ein Anteil an ungelösten Pigmenten zurück, so liegt die Löslichkeit des Pigments unterhalb von 0,5 g/L.

Geeignete Farbpigmente können anorganischen und/oder organischen Ursprungs sein.

In einer bevorzugten Ausführungsform ist ein erfindungsgemäßes Färbemittel (F) dadurch gekennzeichnet, dass es mindestens eine farbgebende Verbindung (F-2) aus der Gruppe der anorganischen und/oder organischen Pigmente enthält.

In einer bevorzugten Ausführungsform ist ein erfindungsgemäßes Färbemittel (F) dadurch gekennzeichnet, dass es mindestens ein anorganisches und/oder organisches Pigment (F-2) enthält.

Bevorzugte Farbpigmente sind ausgewählt aus synthetischen oder natürlichen anorganischen Pigmenten. Anorganische Farbpigmente natürlichen Ursprungs können beispielsweise aus Kreide, Ocker, Umbra, Grünerde, gebranntem Terra di Siena oder Graphit hergestellt werden. Weiterhin können als anorganische Farbpigmente Schwarzpigmente wie z. B. Eisenoxidschwarz, Buntpigmente wie z. B. Ultramarin oder Eisenoxidrot sowie Fluoreszenz- oder Phosphoreszenzpigmente eingesetzt werden.

Besonders geeignet sind farbige Metalloxide, -hydroxide und -oxidhydrate, Mischphasenpigmente, schwefelhaltige Silicate, Silicate, Metallsulfide, komplexe Metallcyanide, Metallsulfate, -chromate und/oder -molybdate. Insbesondere bevorzugte Farbpigmente sind schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510) und/oder Carmine (Cochineal).

Erfindungsgemäß ebenfalls besonders bevorzugte Farbpigmente sind farbige Perlglanzpigmente. Diese basieren üblicherweise auf Mica- und/oder Glimmerbasis und können mit einem oder mehreren Metalloxiden beschichtet sein. Glimmer gehört zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet.

Alternativ zu natürlichem Glimmer kann auch ggfs. mit einem oder mehrere Metalloxide(en) beschichtetes, synthetisches Mica als Perlglanzpigment verwendet werden. Besonders bevorzugte Perlglanzpigmente basieren auf natürlichem oder synthetischem Mica (Glimmer) und sind mit einem oder mehreren der zuvor genannten Metalloxide beschichtet. Die Farbe der jeweiligen Pigmente kann durch Variation der Schichtdicke des oder der Metalloxids(e) variiert werden.

In einer weiteren bevorzugten Ausführungsform ist ein erfindungsgemäßes Verfahren dadurch gekennzeichnet, dass das Färbemittel (F) mindestens anorganisches Pigment (F-2) enthält, das bevorzugt ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

In einer weiteren bevorzugten Ausführungsform ist ein erfindungsgemäßes Verfahren dadurch gekennzeichnet, dass das Färbemittel (F) mindestens ein Pigment enthält, das ausgewählt ist aus Pigmenten auf Mica- oder Glimmerbasis, die mit einem oder mehreren Metalloxiden aus der Gruppe aus Titandioxid (CI 77891), schwarzem Eisenoxid (CI 77499), gelbem Eisenoxid (CI 77492), rotem und/oder braunem Eisenoxid (CI 77491, CI 77499), Manganviolett (CI 77742), Ultramarine (Natrium-Aluminiumsulfosilikate, CI 77007, Pigment Blue 29), Chromoxidhydrat (CI 77289), Chromoxid (CI

77288) und/oder Eisenblau (Ferric Ferrocyanide, CI 77510) beschichtet sind.

Beispiele für besonders geeignete Farbpigmente sind im Handel beispielsweise unter den Handelsbezeichnungen Rona®, Colorona®, Xirona®, Dichrona® und Timiron® von der Firma Merck, Ariabel® und Unipure® von der Firma Sensient, Prestige® von der Firma Eckart Cosmetic Colors und Sunshine® von der Firma Sunstar erhältlich.

Ganz besonders bevorzugte Farbpigmente mit der Handelsbezeichnung Colorona® sind beispielsweise:

Colorona Copper, Merck, MICA, CI 77491 (IRON OXIDES)
Colorona Passion Orange, Merck, Mica, CI 77491 (Iron Oxides), Alumina
Colorona Patina Silver, Merck, MICA, CI 77499 (IRON OXIDES), CI 77891 (TITANIUM DIOXIDE)
Colorona RY, Merck, CI 77891 (TITANIUM DIOXIDE), MICA, CI 75470 (CARMINE)
Colorona Oriental Beige, Merck, MICA, CI 77891 (TITANIUM DIOXIDE), CI 77491 (IRON OXIDES)
Colorona Dark Blue, Merck, MICA, TITANIUM DIOXIDE, FERRIC FERROCYANIDE
Colorona Chameleon, Merck, CI 77491 (IRON OXIDES), MICA
Colorona Aborigine Amber, Merck, MICA, CI 77499 (IRON OXIDES), CI 77891 (TITANIUM DIOXIDE)
Colorona Blackstar Blue, Merck, CI 77499 (IRON OXIDES), MICA
Colorona Patagonian Purple, Merck, MICA, CI 77491 (IRON OXIDES), CI 77891 (TITANIUM DIOXIDE), CI 77510 (FERRIC FERROCYANIDE)
Colorona Red Brown, Merck, MICA, CI 77491 (IRON OXIDES), CI 77891 (TITANIUM DIOXIDE)
Colorona Russet, Merck, CI 77491 (TITANIUM DIOXIDE), MICA, CI 77891 (IRON OXIDES)
Colorona Imperial Red, Merck, MICA, TITANIUM DIOXIDE (CI 77891), D&C RED NO. 30 (CI 73360)
Colorona Majestic Green, Merck, CI 77891 (TITANIUM DIOXIDE), MICA, CI 77288 (CHROMIUM OXIDE GREENS)
Colorona Light Blue, Merck, MICA, TITANIUM DIOXIDE (CI 77891), FERRIC FERROCYANIDE (CI 77510)
Colorona Red Gold, Merck, MICA, CI 77891 (TITANIUM DIOXIDE), CI 77491 (IRON OXIDES)
Colorona Gold Plus MP 25, Merck, MICA, TITANIUM DIOXIDE (CI 77891), IRON OXIDES (CI 77491)
Colorona Carmine Red, Merck, MICA, TITANIUM DIOXIDE, CARMINE
Colorona Blackstar Green, Merck, MICA, CI 77499 (IRON OXIDES)
Colorona Bordeaux, Merck, MICA, CI 77491 (IRON OXIDES)
Colorona Bronze, Merck, MICA, CI 77491 (IRON OXIDES)
Colorona Bronze Fine, Merck, MICA, CI 77491 (IRON OXIDES)
Colorona Fine Gold MP 20, Merck, MICA, CI 77891 (TITANIUM DIOXIDE), CI 77491 (IRON OXIDES)
Colorona Sienna Fine, Merck, CI 77491 (IRON OXIDES), MICA
Colorona Sienna, Merck, MICA, CI 77491 (IRON OXIDES)
Colorona Precious Gold, Merck, Mica, CI 77891 (Titanium dioxide), Silica, CI 77491 (Iron oxides), Tin oxide
Colorona Sun Gold Sparkle MP 29, Merck, MICA, TITANIUM DIOXIDE, IRON OXIDES, MICA, CI 77891, CI 77491 (EU)
Colorona Mica Black, Merck, CI 77499 (Iron oxides), Mica, CI 77891 (Titanium dioxide)
Colorona Bright Gold, Merck, Mica, CI 77891 (Titanium dioxide), CI 77491(Iron oxides)
Colorona Blackstar Gold, Merck, MICA, CI 77499 (IRON OXIDES)
Weiterhin besonders bevorzugte Farbpigmente mit der Handelsbezeichnung Xirona® sind beispielsweise:

Xirona Golden Sky, Merck, Silica, CI 77891 (Titanium Dioxide), Tin Oxide
Xirona Caribbean Blue, Merck, Mica, CI 77891 (Titanium Dioxide), Silica, Tin Oxide
Xirona Kiwi Rose, Merck, Silica, CI 77891 (Titanium Dioxide), Tin Oxide
Xirona Magic Mauve, Merck, Silica, CI 77891 (Titanium Dioxide), Tin Oxide.

Zudem sind besonders bevorzugte Farbpigmente mit der Handelsbezeichnung Unipure® beispielsweise:

Unipure Red LC 381 EM, Sensient CI 77491 (Iron Oxides), Silica
Unipure Black LC 989 EM, Sensient, CI 77499 (Iron Oxides), Silica
Unipure Yellow LC 182 EM, Sensient, CI 77492 (Iron Oxides), Silica

Im Rahmen einer weiteren Ausführungsform kann das erfindungsgemäße Vorbehandlungsmittel (V) auch ein oder mehrere organische Pigmente enthalten

Bei den erfindungsgemäßen organischen Pigmenten handelt es sich um entsprechend unlösliche, organische Farbstoffe oder Farblacke, die beispielsweise aus der Gruppe der Nitroso-, Nitro- Azo-, Xanthen-, Anthrachinon-, Isoindolinon-, Isoindolin-, Chinacridon-, Perinon-, Perylen- , Diketopyrrolopyorrol-, Indigo-, Thioindido-, Dioxazin-, und/oder Triarylmethan-Verbindungen ausgewählt sein können.

Als besonders gut geeignete organische Pigmente können beispielsweise Carmin, Chinacridon, Phthalocyanin,

Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470 genannt werden.

In einer weiteren besonders bevorzugten Ausführungsform ist ein erfindungsgemäßes Verfahren dadurch gekennzeichnet, dass das Färbemittel (F) mindestens ein organisches Pigment (F-2) enthält, das bevorzugt ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

Bei dem organischen Pigment kann es sich weiterhin auch um einen Farblack handeln. Unter der Bezeichnung Farblack wird im Sinn der Erfindung Partikel verstanden, welche eine Schicht aus absorbierten Farbstoffen umfassen, wobei die Einheit aus Partikel und Farbstoff unter den o.g. Bedingungen unlöslich ist. Bei den Partikeln kann es sich beispielsweise um anorganische Substrate handeln, die Aluminium, Silica, Calciumborosilkat, Calciumaluminiumborosilikat oder auch Aluminium sein können.

Als Farblack kann beispielsweise der Alizarin-Farblack eingesetzt werden.

Aufgrund ihrer ausgezeichneten Licht- und Temperaturbeständigkeit ist die Verwendung der zuvor genannten Pigmente in dem Färbemittel (F) des erfindungsgemäßen Verfahrens ganz besonders bevorzugt. Weiterhin ist es bevorzugt, wenn die eingesetzten Pigmente eine bestimmte Teilchengröße aufweisen. Es ist daher erfindungsgemäß vorteilhaft, wenn das mindestens eine Pigment eine mittlere Teilchengröße $D_{50}$ von 1,0 bis 50 $\mu$m, vorzugsweise von 5,0 bis 45 $\mu$m, bevorzugt von 10 bis 40 $\mu$m, insbesondere von 14 bis 30 $\mu$m, aufweist. Die mittlere Teilchengröße $D_{50}$ kann beispielsweise unter Verwendung von dynamischer Lichtstreuung (DLS) bestimmt werden.

Das bzw. die Pigmente (F-2) stellen den zweiten wesentlichen Bestandteil des erfindungsgemäßen Färbemittels (F) dar und werden bevorzugt in bestimmten Mengenbereichen im Mittel eingesetzt. Besonders gute Ergebnisse wurden erhalten, wenn das Färbemittel - bezogen auf das Gesamtgewicht des Färbemittels - ein oder mehrere Pigmente (F-2) in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthielt.

In einer weiteren ganz besonders bevorzugten Ausführungsform ist ein erfindungsgemäßes Verfahren dadurch gekennzeichnet, dass das Färbemittel (F) - bezogen auf das Gesamtgewicht des Färbemittels - ein oder mehrere Pigmente (F-2) in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthält.

Direktziehende Farbstoffe im Färbemittel (F)

Prinzipiell können die im erfindungsgemäßen Verfahren angewendeten Färbemittel (F) auch einen oder mehrere direktziehende Farbstofe als optionale Bestandteile enthalten. Bei direktziehenden Farbstoffen handelt es sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone, Triarylmethanfarbstoffe oder Indophenole.

Die direktziehenden Farbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die direktziehenden Farbstoffe im Sinne der vorliegenden Erfindung eine Löslichkeit in Wasser (760 mmHg) bei 25 °C von mehr als 1,0 g/L.

Der wesentliche Vorteil des erfindungsgemäßen Verfahrens liegt jedoch darin, dass die mit dem Färbemittel (F) auf Pigment-Basis erzielbaren Färbungen einerseits sehr waschstabil sind, andererseits aber auch eine sehr hohe Nuancenstabilität besitzen. Dies bedeutet, dass das Verblassen der Färbung, sofern es nach mehreren Wäschen des Keratinmaterials in verringertem Ausmaß auftritt, unter Erhalt der Farbnuance ohne sichtbare Farbverschiebung erfolgt. Diese Nuancenstabilität ist selbst dann zu beobachten, wenn das Färbemittel (F) eine Mischung aus Pigmenten (F-2) verschiedener Farbe enthält.

Ohne auf diese Theorie festgelegt zu sein, wird in diesem Zusammenhang vermutet, dass die Ursache für die hohe Stabilität der Farbnuance darin zu sehen ist, dass alle Pigmente sich in Form eines Films an der Oberfläche des Keratinmaterials ablagern. Im Gegensatz zu direktziehenden Farbstoffen können die Pigmente nicht in das Keratinmaterial hinein diffundieren, und ebenfalls im Gegensatz zu direktziehenden Farbstoffen kann die Größe bzw. Struktur

eines Pigments so auch nicht dessen Eindringtiefe in das Keratinmaterial hinein beeinflussen.

Wird ein Gemisch aus direktziehenden Farbstoffen verschiedener Farbe auf das Keratinmaterial appliziert, so liegen diesen verschiedenen Farbstoffen in der Regel verschiedene chromophore Strukturen und Moleküle verschiedener Größe zu Grunde. Aufgrund ihrer strukturellen Unterschiede können diese veschiedenen Farbstoffe unterschiedlich tief in das Keratinmaterial hinein diffundieren und werden während der Wäsche auch in unterschiedlich starkem Maß wieder aus dem Keratinmaterial herausgewaschen. Inbesondere bei Färbungen in Naturtönen, die beispielsweise durch Einsatz eines Gemisches aus einem gelben, einem roten und einem blauen direktziehenden Farbstoff erzeugt werden, ist so im Verlauf mehrere Wäschen oder Shampoonierungen eine Farbverschiebung vom Braunen ins gelbliche, rötliche oder bläuliche zu beobachten.

Die über das erfindungsgemäße Verfahren erzeugten Färbungen beruhen auf einem Pigment-Silikon-Film, der an der Oberfläche des Keratinmaterials lokalisiert ist. Waschversuche haben nun gezeigt, dass wiederholte Wäschen zwar zu einer geringfügigen Verringerung der Farbintensität führen, hierbei jedoch keine Verschiebung des Farbtons auftritt. Das bei einer Haarwäsche erfolgende Herauslösen verschiedenfarbiger Pigmente aus dem Film erfolgt demnach wesentlich gleichmäßiger.

Dass diese Farbverschiebung bei Anwendung des erfindungsgemäßen Verfahrens nicht auftritt, ist ein wesentlicher Vorteil gegenüber einem auf direktziehenden Farbstoffen beruhenden Färbesystem. Aus diesem Grund ist es ganz besonders bevorzugt, wenn das Färbemittel (F) keine direktziehenden Farbstoffe enthält oder aber diese in nur sehr geringen Mengen beinhaltet.

Im Rahmen eine weiteren, ganz besonders bevorzugten Ausführungsform ist ein erfindungsgemäßes Verfahren dadurch gekennzeichnet, dass die Gesamtmenge der im Färbemittel (F) enthaltenen direktziehenden Farbstoffe - bezogen auf das Gesamtgewicht des Färbemittels (F) - bei einem Wert unterhalb von 0,1 Gew.-%, bevorzugt unterhalb von 0,05 Gew.-%, weiter bevorzugt unterhalb von 0,01 Gew.-% und ganz besonders bevorzugt unterhalb von 0,001 Gew-% liegt.

In anderen Worten ist in einer weiteren ganz besonders bevorzugten Ausführungsform ein erfindungsgemäßes Verfahren dadurch gekennzeichnet, dass die Gesamtmenge der im Färbemittel (F) enthaltenen direktziehenden Farbstoffe - bezogen auf das Gesamtgewicht des Färbemittels (F) - bei einem Wert unterhalb von 0,1 Gew.-%, bevorzugt unterhalb von 0,05 Gew.-%, weiter bevorzugt unterhalb von 0,01 Gew.-% und ganz besonders bevorzugt unterhalb von 0,001 Gew-% liegt, wobei die direktziehenden Farbstoffe dadurch gekennzeichnet sind, dass sie eine Löslichkeit in Wasser (760 mmHg) bei 25 °C von mehr als 0,5 g/L besitzen.

Im Rahmen eine weiteren, ganz besonders bevorzugten Ausführungsform ist ein erfindungsgemäßes Verfahren dadurch gekennzeichnet, dass das Färbemittel (F) frei ist von direktziehenden Farbstoffen.

Direktziehende Farbstoffe können in anionische, kationische und nichtionische direktziehende Farbstoffe unterteilt werden.

Kationische direktziehende Farbstoffe sind beispielsweise Basic Blue 7, Basic Blue 26, HC Blue 16, Basic Violet 2 und Basic Violet 14, Basic Yellow 57, Basic Red 76, Basic Blue 16, Basic Blue 347 (Cationic Blue 347 / Dystar), HC Blue No. 16, Basic Blue 99, Basic Brown 16, Basic Brown 17, Basic Yellow 57, Basic Yellow 87, Basic Orange 31, Basic Red 51 Basic Red 76.

Als nichtionische direktziehende Farbstoffe können beipsielsweise nichtionische Nitro- und Chinonfarbstoffe und neutrale Azofarbstoffe genannt werden. Beispiele für nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12,

Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Anionische direktziehende Farbstoffe werden auch als Säurefarbstoffe bezeichnet. Unter Säurefarbstoffen werden direktziehende Farbstoffe verstanden, die mindestens eine Carbonsäuregruppierung (-COOH) und/oder eine Sulfonsäuregruppierung ($-SO_3H$) besitzen. In Abhängigkeit vom pH-Wert liegen die protonierten Formen (-COOH, $-SO_3H$) der Carbonsäure- bzw. Sulfonsäuregruppierungen mit ihren deprotonierten Formen ($-COO^-$, $-SO_3^-$ vor) im Gleichgewicht vor. Mit abnehmendem pH-Wert steigt der Anteil der protonierten Formen. Werden direktziehende Farbstoffe in Form ihrer Salze eingesetzt, so liegen die Carbonsäuregruppen bzw. Sulfonsäuregruppen in deprotonierter Form vor und sind zur Einhaltung der Elektroneutralität mit entsprechenden stöchiometrischen Äquivalente an Kationen neutralisiert. Erfindungsgemäße Säurefarbstoffe können auch in Form ihrer Natriumsalze und/oder ihrer Kaliumsalze eingesetzt werden.

Die Säurefarbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die Säurefarbstoffe im Sinne der vorliegenden Erfindung besitzen eine Löslichkeit in Wasser (760 mmHg) bei 25 °C von mehr als 1,0 g/L.

Die Erdalkalisalze (wie beispielsweise Calciumsalze und Magnesiumsalze) bzw. Aluminiumsalze von Säurefarbstoffen besitzen oftmals eine schlechtere Löslichkeit als die entsprechenden Alkalisalze. Sofern die Löslichkeit dieser Salze unterhalb von 0,5 g/L (25 °C, 760 mmHg) liegt, fallen diese nicht unter die Definition eines direktziehenden Farbstoffes.

Ein wesentliches Merkmal der Säurefarbstoffe ist ihr Vermögen, anionische Ladungen auszubilden, wobei die hierfür verantwortlichen Carbonsäure- bzw. Sulfonsäuregruppen üblicherweise an verschiedene chromophore Systeme geknüpft sind. Geeignete chromophore Systeme finden sich beispielsweise in den Strukturen von Nitrophenylendiaminen, Nitroaminophenolen, Azofarbstoffen, Anthrachinonfarbstoffen, Triarylmethanfarbstoffen, Xanthen-Farbstoffen, Rhodamin-Farbstoffen, Oxazinfarbstoffen und/oder Indophenolfarbstoffen.

Als Beispiele für Säurefarbstoffe können können genannt werden: Acid Yellow 1 (D&C Yellow 7, Citronin A, Ext. D&C Yellow No. 7, Japan Yellow 403,CI 10316, COLIPA n° B001), Acid Yellow 3 (COLIPA n° : C 54, D&C Yellow N° 10, Quinoline Yellow, E104, Food Yellow 13), Acid Yellow 9 (CI 13015), Acid Yellow 17 (CI 18965), Acid Yellow 23 (COLIPA n° C 29, Covacap Jaune W1100 (LCW), Sicovit Tartrazine 85 E 102 (BASF), Tartrazine, Food Yellow 4, Japan Yellow 4, FD&C Yellow No. 5), Acid Yellow 36 (CI 13065), Acid Yellow 121 (CI 18690), Acid Orange 6 (CI 14270), Acid Orange 7 (2-Naphthol orange, Orange II, CI 15510, D&C Orange 4, COLIPA n° C015), Acid Orange 10 (C.I. 16230; Orange G sodium salt), Acid Orange 11 (CI 45370), Acid Orange 15 (CI 50120), Acid Orange 20 (CI 14600), Acid Orange 24 (BROWN 1;CI 20170;KATSU201;nosodiumsalt;Brown No.201;RESORCIN BROWN;ACID ORANGE 24;Japan Brown 201;D & C Brown No.1), Acid Red 14 (C.I.14720), Acid Red 18 (E124, Red 18; CI 16255), Acid Red 27 (E 123, CI 16185, C-Rot 46, Echtrot D, FD&C Red Nr.2, Food Red 9, Naphtholrot S), Acid Red 33 (Red 33, Fuchsia Red, D&C Red 33, CI 17200), Acid Red 35 (CI C.I.18065), Acid Red 51 (CI 45430, Pyrosin B, Tetraiodfluorescein, Eosin J, Iodeosin), Acid Red 52 (CI 45100, Food Red 106, Solar Rhodamine B, Acid Rhodamine B, Red n° 106 Pontacyl Brilliant Pink), Acid Red 73 (CI CI 27290), Acid Red 87 (Eosin, CI 45380), Acid Red 92 (COLIPA n° C53, CI 45410), Acid Red 95 (CI 45425, Erythtosine,Simacid Erythrosine Y), Acid Red 184 (CI 15685), Acid Red 195, Acid Violet 43 (Jarocol Violet 43, Ext. D&C Violet n° 2, C.I. 60730, COLIPA n° C063), Acid Violet 49 (CI 42640), Acid Violet 50 (CI 50325), Acid Blue 1 (Patent Blue, CI 42045), Acid Blue 3 (Patent Blau V, CI 42051), Acid Blue 7 (CI 42080), Acid Blue 104 (CI 42735), Acid Blue 9 (E 133, Patentblau AE, Amidoblau AE, Erioglaucin A, CI 42090, C.I. Food Blue 2), Acid Blue 62 (CI 62045), Acid Blue 74 (E 132, CI 73015), Acid Blue 80 (CI 61585), Acid Green 3 (CI 42085, Foodgreen1), Acid Green 5 (CI 42095), Acid Green 9 (C.I.42100), Acid Green 22 (C.I.42170), Acid Green 25 (CI 61570, Japan Green 201, D&C Green No. 5), Acid Green 50 (Brillantsäuregrün BS, C.I. 44090, Acid Brilliant Green BS, E 142), Acid Black 1 (Black n° 401, Naphthalene Black 10B, Amido Black 10B, CI 20 470, COLIPA n° B15), Acid Black 52 (CI 15711), Food Yellow 8 (CI 14270), Food Blue 5, D&C Yellow 8, D&C Green 5, D&C Orange 10, D&C Orange 11, D&C Red 21, D&C Red 27, D&C Red 33, D&C Violet 2 und/oder D&C Brown 1.

Die Wasserlöslichkeit der anionischen direktziehenden Farbstoffe kann beispielsweise auf dem folgenden Weg bestimmt werden. 0,1 g des anionischen direktziehenden Farbstoffes werden in ein Becherglas gegeben. Es wird ein Rührfisch hinzugegeben. Dann werden 100 ml Wasser hinzugefügt. Diese Mischung wird auf einem Magnetrührer unter Rühren auf 25 °C erwärmt. Es wird für 60 Minuten gerührt. Danach wird die wässrige Mischung visuell beurteilt. Sind noch ungelöste Reste vorhanden, wird die Wassermenge - beispielsweise in Schritten von 10 ml - erhöht. Es wird solange Wasser zugegeben, bis sich die eingesetzte Farbstoffmenge komplett gelöst hat. Sofern sich die Farbstoff-Wasser-Mischung aufgrund der hohen Intenstität des Farbstoffes nicht visuell beurteilten lässt, wird die Mischung filtriert. Bleibt auf dem Filterpapier ein Anteil an ungelösten Farbstoffen zurück, wird der Löslichkeitsversuch mit einer höheren Wassermenge wiederholt. Lösen sich 0,1 g des anionischen direktziehenden Farbstoffes bei 25 °C in 100 ml Wasser, so liegt die Löslichkeit des Farbstoffes bei 1,0 g/L.

Acid Yellow 1 trägt den Namen 8-Hydroxy-5,7-dinitro-2-naphthalenesulfonsäure Dinatriumsalz und besitzt eine Löslichkeit in Wasser von mindestens 40 g/L (25°C).

Acid Yellow 3 ist ein Gemisch der Natriuimsalze von Mono- und Sisulfonsäuren von 2-(2-Chinolyl)-1H-indene-1,3(2H)-dion und besitzt eine Wasserlöslichkeit von 20 g/L (25 °C).

Acid Yellow 9 ist das Dinatriumsalz der 8-Hydroxy-5,7-dinitro-2-naphthalenesulfonsäure, seine Wasserlöslichkeit liegt oberhalb von 40 g/L (25 °C).

Acid Yellow 23 ist das Trinatriumsalz der4,5-Dihydro-5-oxo-1-(4-sulfophenyl)-4-((4-sulfophenyl)azo)-1H-pyrazole-3-carbonsäure und bei 25 °C gut in Wasser löslich.

Acid Orange 7 ist das Natriumsalz des 4-[(2-Hydroxy-1-naphthyl)azo]benzensulfonats. Seine Wasserlöslichkeit beträgt mehr als 7 g/L (25 °C).

Acid Red 18 ist das Trinatirumsalz von 7-Hydroxy-8-[(E)-(4-sulfonato-1-naphthyl)-diazenyl)]-1,3-naphthalenedisulfonat und besitzt eine sehr hohe Wasserlöslichkeit von mehr als 20 GEw.-%.

Acid Red 33 ist das Diantriumsalz des 5-Amino-4-hydroxy-3-(phenylazo)-naphthalene-2,7-disulphonats, seine Wasserlöslichkeit liegt bei 2,5 g/L (25 °C).

Bei Acid Red 92 handelt es sich um das Dinatriumsalz der 3,4,5,6-Tetrachloro-2-(1 ,4,5,8-tetrabromo-6-hydroxy-3-oxoxanthen-9-yl)benzoesäure, dessen Wasserlöslichkeit mit größer 10 g/L angegeben wird (25 °C).

Acid Blue 9 ist das Dinatriumsalz von 2-({4-[N-ethyl(3-sulfonatobenzyl]amino]phenyl}{4-[(N-ethyl(3-sulfonatobenzyl)imino]-2,5-cyclohexadien-1-ylidene}methyl)-benzenesulfonat und besitzt eine Wasserlöslichkeit von mehr als 20 Gew.-% (25 °C).

<u>weitere optionale Inhalttstoffe in den Mitteln (V) und/oder (F)</u>

Zusätzlich zu den bereits beschriebenen erfindungswesentlichen Bestandteilen können das Vorbehandlungsmittel (V) und/oder das Färbemittel (F) auch noch weitere optionale Inhaltsstoffe enthalten.

So können die Mittel ferner auch noch weitere Wirk-, Hilfs- und Zusatzstoffe enthalten, wie beispielsweise Lösungsmittel, Fettbestandteile wie beispielsweise der $C_8$-$C_{30}$-Fettalkohole, der $C_8$-$C_{30}$-Fettsäuretriglyceride, der $C_8$-$C_{30}$-Fettsäuremonoglyceride, der $C_8$-$C_{30}$-Fettsäurediglyceride und/oder der Kohlenwasserstoffe; Polymere; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; sowie Treibmittel wie Propan-Butan-Gemische, $N_2O$, Dimethylether, $CO_2$ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Zubereitungen bevorzugt in Mengen von jeweils 0,0001 bis 25 Gew.-%, insbesondere von 0,0005 bis 15 Gew.-%, bezogen auf das Gesamtgewicht des jeweiligen Mittels, eingesetzt.

<u>pH-Wert von Vorbehandlungsmittel (V) und/oder Färbemittel (F)</u>

Die pH-Werte der erfindungsgemäßen Mittel (V) und (F) können auf einen leicht sauren bis alkalischen pH-Wert eingestellt werden.

Im Rahmen einer Ausführungsform besitzt das Vorbehandlungsmittel (V) einen pH-Wert von 6,0 bis 12,0, bevorzugt von 7,0 bis 11,5 weiter bevorzugt von 7,5 bis 11,0 und ganz besonders bevorzugt von 9,0 bis 11,0. Wurden Vorbehandlungsmittel (V) mit diesen pH-Werten im erfindungsgemäßen Verfahren eingesetzt, so ließen sich Färbungen mit besonders guten Echtheitseigenschaften erzielen.

Im Rahmen einer weiteren Ausführungsform ist ein erfindungsgemäßes Verfahren dadurch gekennzeichnet dass das Vorbehandlungsmittel (V) einen pH-Wert von 6,0 bis 12,0, bevorzugt von 7,0 bis 11,5 weiter bevorzugt von 7,5 bis 11,0 und ganz besonders bevorzugt von 9,0 bis 11,0 besitzt.

Die pH-Werte des erfindungsgemäßen Färbemittels (F) können auf einen leicht sauren bis alkalischen pH-Wert eingestellt werden. Ganz besonders bevorzugt besitzt das Färbemittel (F) einen pH-Wert im Bereich von 5,0 bis 10,0, bevorzugt von 6,0 bis 9,5, weiter bevorzugt von 6,0 bis 8,7 und ganz besonders bevorzugt von 6,0 bis 7,5.

Zur Einstellung der jeweiligen gewünschten pH-Werte können die dem Fachmann bekannten Alkalisierungsmittel und Acidifizierungsmittel verwendet werden. Bei den pH-Werten im Sinne der vorliegenden Erfindung handelt es sich um pH-Werte, die bei einer Temperatur von 22°C gemessen wurden.

Als Alkalisierungsmittel können die Mittel beispielsweise Ammoniak, Alkanolamine und/oder basische Aminosäuren enthalten.

Die in dem erfindungsgemäßen Mittel einsetzbaren Alkanolamine werden bevorzugt ausgewählt aus primären Aminen mit einem $C_2$-$C_6$-Alkylgrundkörper, der mindestens eine Hydroxylgruppe trägt. Bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol,

2-Amino-2-methylpropan-1,3-diol.

Erfindungsgemäß besonders bevorzugte Alkanolamine werden ausgewählt aus 2-Aminoethan-1-ol und/oder 2-Amino-2-methylpropan-1-ol. Eine besonders bevorzugte Ausführungsform ist daher dadurch gekennzeichnet, dass das erfindungsgemäße Mittel als Alkalisierungsmittel ein Alkanolamin ausgewählt aus 2-Aminoethan-1-ol und/oder 2-Amino-2-methylpropan-1-ol enthält.

Als Aminosäure im Sinne der Erfindung gilt eine organische Verbindung, die in ihrer Struktur mindestens eine protonierbare Aminogruppe und mindestens eine -COOH- oder eine -$SO_3$H-Gruppe enthält. Bevorzugte Aminosäuren sind Aminocarbonsäuren, insbesondere $\alpha$-(alpha)-Aminocarbonsäuren und $\omega$-Aminocarbonsäuren, wobei $\alpha$-Aminocarbonsäuren besonders bevorzugt sind.

Unter basischen Aminosäuren sind erfindungsgemäß solche Aminosäuren zu verstehen, welche einen isoelektrischen Punkt pl von größer 7,0 besitzen.

Basische $\alpha$-Aminocarbonsäuren enthalten mindestens ein asymmetrisches Kohlenstoffatom. Im Rahmen der vorliegenden Erfindung können beide möglichen Enantiomere als spezifische Verbindung oder auch deren Gemische, insbesondere als Racemate, gleichermaßen eingesetzt werden. Es ist jedoch besonders vorteilhaft, die natürlich bevorzugt vorkommende Isomerenform, üblicherweise in L-Konfiguration, einzusetzen.

Die basischen Aminosäuren werden bevorzugt ausgewählt aus der Gruppe, die gebildet wird aus Arginin, Lysin, Ornithin und Histidin, besonders bevorzugt aus Arginin und Lysin. In einer weiteren besonders bevorzugten Ausführungsform ist ein erfindungsgemäßes Mittel daher dadurch gekennzeichnet, dass es sich bei dem Alkalisierungsmittel um eine basische Aminosäure aus der Gruppe Arginin, Lysin, Ornithin und/oder Histidin handelt.

Darüber hinaus kann das Mittel weitere Alkalisierungsmittel, insbesondere anorganische Alkalisierungsmittel enthalten. Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Ebenfalls erfindungsgemäß ist die Einstellung des gewünschten pH-Werte über ein Puffersystem. Unter einem Puffer oder einem Puffersystem wird üblicherweise ein Gemisch aus einer schwachen oder mittelstarken Säure (z. B. Essigsäure) mit einem praktisch völlig dissoziierten Neutralsalz derselben Säure (z. B. Natriumacetat) verstanden. Wird etwas Base oder Säure zugegeben, so ändert sich der pH-Wert kaum (Pufferung). Die Wirkung der in einer Pufferlösung enthaltenen Puffersubstanzen beruht auf der Abfangreaktion von Wasserstoff- bzw. Hydroxid-Ionen unter Bildung schwacher Säuren bzw. Basen aufgrund ihres Dissoziationsgleichgewichts. Ein Puffersystem kann aus einem Gemisch einer anorganischen oder organischen Säure und einem korrespondierenden Salz dieser Säure ausgebildet werden. Säuren können gepuffert werden durch alle Salze aus schwachen Säuren und starken Basen, Basen durch Salze aus starken Säuren und schwachen Basen. Die starke (vollständig in Ionen dissoziierte) Salzsäure kann z. B. durch Zusatz von Natriumacetat abgepuffert werden. Entsprechend dem Gleichgewicht

$$H_3C-COONa + HCl \rightleftharpoons NaCl + H_3C-COOH$$

wird Salzsäure durch Natriumacetat unter Bildung von Kochsalz in die schwache Essigsäure übergeführt, die in Gegenwart eines Natriumacetat-Überschusses nur zu einem sehr geringen Anteil dissoziiert. Puffer, die sowohl gegenüber Säuren als auch Basen wirken, sind Gemische aus schwachen Säuren und ihren Salzen.

Literaturbekannte Beispiele für Puffersysteme sind Essigsäure/Natriumacetat, Borsäure/Natriumborat, Phosphorsäure/Natriumphosphat und Hydrogencarbonat/Soda.

Der pH-Wert des erfindungsgemäßen Mittels kann zum Beispiel durch Zusatz eines anorganischen oder organischen Puffersystems eingestellt werden. Unter einem anorganischen Puffersystem wird im Sinne der vorliegenden Erfindung eine Mischung aus einer anorganischen Säure und ihrer konjugierten korrespondieren anorganischen Base verstanden.

Unter einem organischen Puffersystem wird im Sinne der vorliegenden Erfindung eine Mischung aus einer organischen Säure und ihrer konjugierten korrespondieren Base verstanden. Bedingt durch den organischen Säure-Rest ist auch die konjugierte korrespondierende Base der organischen Säure ebenfalls organisch. Hierbei kann das zur Neutralisation der Ladung des Säure-Anions vorhandene Kation anorganisch oder organisch sein.

Beispiele für anorganische Säuren sind Schwefelsäure, Salzsäure und Phosphorsäure ($H_3PO_4$). Bei Phosphorsäure handelt es sich um eine mittelstarke Säure, die ganz besonders bevorzugt ist.

Eine ganz besonders gut geeignete anorganische Säure ist Kaliumdihydrogenphosphat Kaliumdihydrogenphosphat besitzt die Summenformel $KH_2PO_4$ und trägt die CAS-Nummer 7778-77-0. Kaliumdihydrogenphosphat besitzt eine molare Masse von 136,09 g/mol. Es ist gut löslich in Wasser (222 g/l bei 20 °C) und reagiert in Wasser sauer. Eine 5 % ige Lösung von Kaliumdihydrogenphosphat in Wasser besitzt einen pH-Wert von 4,4.

Eine weitere ganz besonders gut geeignete anorganische Säure ist Natriumdihydrogenphosphat. Natriumdihydrogenphosphat besitzt die Summenformel $NaH_2PO_4$ und trägt die CAS-Nummern 7558-80-7 (Anhydrat), 10049-21-5

(Monohdrat) und 13472-35-0 (Dihydrat). Das wasserfreie Natriumdihydrogenphosphat besitzt eine molare Masse von 119,98 g/mol. Natriumdihydrogenphosphat reagiert in wässriger Lösung sauer.

Besonders bevorzugt als korrespondierendes Salz der vorgenannten beiden Säuren ist Dikaliumhydrogenphosphat. Dikaliumhydrogenphosphat besitzt die Summenformel $K_2HPO_4$ und trägt die CAS-Nummern 7758-11-4 (wasserfrei) und 16788-57-1 (Trihydrat). Das wasserfreie Dikaliumhydrogenphosphat besitzt eine molare Masse von 174,18 g/mol. Dikaliumhydrogenphosphat reagiert in wässriger Lösung alkalisch.

Ebenfalls besonders bevorzugt als korresprondierendens Salz der vorgenannten beiden Säuren ist Dinatriumhydrogenphosphat. Dinatriumhydrogenphosphat besitzt die Summenformel $Na_2HPO_4$ und trägt die CAS-Nummern 7558-79-4 (wasserfrei), 10028-24-7 (Dihydrat), 7782-85-6 (Heptahydrat) und 10039-32-4 (Dodecahydrat). Das wasserfreie Dinatriumhydrogenphosphat besitzt eine molare Masse von 141,96 g/mol. Dinatriumhydrogenphosphat reagiert in wässriger Lösung alkalisch.

Beispiele für organische Säuren sind Zitronensäure, Bernsteinsäure, Weinsäure, Milchsäure, Essigsäure, Äpfelsäure, Malonsäure und Maleinsäure.

Beispiele für die korrespondierenden Salze dieser organischen Säuren sind die Natrium- und Kaliumsalze der Zitronensäure, die Natrium- und Kaliumsalze der Bernsteinsäure, die Natrium- und Kaliumsalze der Weinsäure, die Natrium- und Kaliumsalze der Milchsäure, die Natrium- und Kaliumsalze der Essigsäure, die Natrium- und Kaliumsalze der Äpfelsäure, die Natrium- und Kaliumsalze der Malonsäure und die die Natrium- und Kaliumsalze der Maleinsäure.

<u>Abfolge der Verfahrensschritte</u>

Wie bereits zuvor beschrieben, wird das Vorbehandlungsmittel (V) vor der Anwendung des Färbemittels (F) appliziert. Das Vorbehandlungsmittel (V) wird auf dem Keratinmaterial angewendet, für einen bestimmten Zeitraum einwirken gelassen und danach wieder mit Wasser ausgespült.

Das Verfahren zur Färbung von keratinischen Fasern, insbesondere menschlichen Haaren, umfasst die folgenden Schritte in der angegebenen Reihenfolge:

(1) Applizieren eines Vorbehandlungsmittels (V) auf die keratinischen Fasern, wobei das Vorbehandlungsmittel (V) bei der Beschreibung des ersten Erfindungsgegenstands im Detail offenbart wurde,
(2) Einwirken des in Schritt (1) applizierten Vorbehandlungsmittels auf die keratinischen Fasern für einen Zeitraum von 2 bis 45 Minuten, bevozugt von 2 bis 30 Minuten und besonders bevorzugt von 2 bis 20 Minuten,
(3) Ausspülen des Vorbehandlungsmittels mit Wasser,
(4) Applizieren eines Färbemittels (F) auf die keratinischen Fasern, wobei das Färbemittel bei der Beschreibung des ersten Erfindungsgegenstands im Detail offenbart wurde,
(5) Einwirken des in Schritt (4) applizierten Färbemittels auf die keratinischen Fasern für einen Zeitraum von 15 Sekunden bis 45 Minuten, bevozugt von 30 Sekunden bis 30 Minuten und besonders bevorzugt von 1 bis 15 Minuten, und
(6) Ausspülen des Färbemittels mit Wasser.

In Schritt (1) des erfindungsgemäßen Verfahrens wird ein Vorbehandlungsmittel (V), das in einem wasserhaltigen Träger mindestens ein Oxidationsmittel enthält, auf die Haare appliziert.

Im darauffolgenden Schritt wird das zuvor applizierte Vorbehandlungsmittel (V) auf die Keratinfasern einwirken gelassen. In diesem Zusammenhang sind verschiedene Einwirkzeiten von 2 bis 45 Minuten, bevozugt von 2 bis 30 Minuten und besonders bevorzugt von 2 bis 20 Minuten möglich.

Im Anschluss an das Einwirken des Vorbehandlungsmittels (V) auf die Keratinfasern wird dieses schließlich in Schritt (3) mit Wasser ausgespült. Hierbei kann das Vorbehandlungsmittel (V) entweder nur mit Wasser, d.h. ohne Zuhilfenahme eines Shampoos, ausgewaschen werden, oder aber der Auswaschvorgang wird durch Anwendung eines Shampoos unterstützt.

Es kann jedoch bevorzugt sein, dass zwischen der Anwendung der beiden Mittel (V) und (F) keine weiteren Mittel, wie beispielsweise andere Conditioner, Stylingmittel appliziert werden. Auf diese Weise wird der maximale Zeitraum, der zwischen der Anwendung der beiden Mittel (V) und (F) liegt, bevorzugt auf ein zeitliches Intervall von maximal 3 Stunden begrenzt.

Zwischen dem Ausspülen des Vorbehandlungsmittels (V) mit Wasser und dem Applizieren eines Färbemittels (F) auf die keratinischen Fasern liegt ein Zeitraum von maximal 3 Stunden liegt.

Das erfindungsgemäße Verfahren ist dadurch gekennzeichnet, dass zwischen den Schritten (3) und (4) ein Zeitraum von maximal 3 Stunden liegt.

Im Rahmen einer weiteren bevorzugten Ausführungsform ist ein erfindungsgemäßes Verfahren dadurch gekennzeichnet, dass Schritt (4) direkt im Anschluss an Schritt (3) erfolgt. Mit Schritt (4) erfolgt die Anwendung des Färbemittels.

Das Einwirken des Färbemittels (F) auf den keratinischen Fasern in Schritt (5) kann beispielsweise für einen Zeitraum von 15 Sekunden bis 30 Minuten, bevorzugt für einen Zeitraum von 30 Sekunden bis 15 Minuten, besonderes bevorzugt für einen Zeitraum von 1 bis 15 Minuten, erfolgen.

Danach wird das Färbemittel (F) schließlich in Schritt (6) mit Wasser ausgespült. Hierbei wird das Färbemittel (F) in einer bevorzugten Ausführungsform nur mit Wasser, d.h. ohne Zuhilfenahme eines nicht erfindungsgemäßen Nachbehandlungsmittels oder eines Shampoos, ausgewaschen.

Beispiele

1. Formulierungen

Es wurde das folgende anwendungsbereite Vorbehandlungsmittel (V) hergestellt (alle Angaben, sofern nichts anderes angegeben ist, in Gew.-%):

| Vorbehandlungsmittel (V) | (V) |
|---|---|
| Natriumsilikat | 14,4 |
| Magnesiumcarbonat | 5,2 |
| Natriumhexametaphosphat | 0,08 |
| Degalan RG S hv (Evonic, Methyl methacrylate, methacrylic acid copolymer, INCI ACRYLATES COPOLYMER) | 0,4 |
| EDTA, Dinatriumsalz | 0,24 |
| Polyquaternium-4 | 0,2 |
| Aerosil 50 (Evonic, hydrophilic fumed silica) | 0,16 |
| Kaliumperoxodisulfat | 12,8 |
| Ammoniumperoxodisulfat | 4,0 |
| Dimethicone | 0,6 |
| Paraffinum Liquidum | 2,7 |
| Natriumbenzoat | 0,24 |
| Dipicolinsäure | 0,06 |
| Dinatriumpyrophosphat | 0,06 |
| Kaliumhydroxid (50 %ige wässrige Lösung) | 0,114 |
| 1,2-Propandiol | 0,3 |
| Etidronsäure (60%ige wässrige Lösung) | 0,15 |
| Cetearylalkohol | 2,16 |
| Ceteareth-20 | 0,72 |
| Wasserstoffperoxid (50%ige wässrige Lösung) | 11,0 |
| Wasser | ad 100 |

Es wurde das folgende Färbemittel (F) hergestellt (alle Angaben, sofern nichts anderes angegeben ist, in Gew.-%):

| Färbemittel (F) | F |
|---|---|
| Emulgade CM (BASF,Cetearyl Isononanoate, Ceteareth-29, Cetearyl alcohol, Glcerylstearate, Glycerin, Ceteareth-10, Cetyl palmitate | 0,6 |
| Cetylalkohol | 0,6 |
| Stearylalkohol | 0,6 |

(fortgesetzt)

| Färbemittel (F) | F |
|---|---|
| Phenoxyethanol | 0,9 |
| Natriumsalicylat | 0,4 |
| 1,2-Propandiol | 2,0 |
| Kaliumdihydrogenphosphat | 0,34 |
| Dinatriumhydrogenphosphat | 0,72 |
| Xanthan Gum | 1,6 |
| Unipure Red LC3071, organisches Pigment CI 15850 | 1,0 g |
| Dow Corning 2-8566 (Siloxanes and Silicones, 3-[(2-Aminoethyl)amino]-2-methylpropyl Me, Di-Me-Siloxane) | 1,0 g |
| Wasser | ad 100 |

2. Anwendung auf Strähnen

Zunächst wurde auf Haarsträhnen (Firma Kerling) das Vorbehandlungsmittel (V) appliziert. Hierzu wurden jeweils 0,2 g Vorbehandlungsmittel (V) pro Gramm Haar auf die Strähnen gegeben, einmassiert und bei Raumtemperatur für 30 Minuten einwirken gelassen. Anschließend wurden die Strähnen mit Wasser ausgespült. Auf die noch feuchten Haare wurde direkt im Anschluss daran das Färbemittel (F) appliziert. Hierzu wurden jeweils 0,2 g Färbemittel (F) pro Gramm Haarsträhne einmassiert, für 1 Minute einwirken gelassen und anschließend wieder mit Wasser ausgespült und getrocknet.

Eine Referenzsträhne wurde ohne Anwendung des Vorbehandlungsmittels (V) direkt mit dem Färbemittel (F) behandelt. Vor Auftrag des Färbemittels (F) wurde die Referenzsträhne nur mit Wasser angefeuchtet, dann erfolgte der Auftrag des Färbemittels nach dem oben beschriebenen Procedere.

Die nun getrockneten Haarsträhnen wurden farbmetrisch mit einem Farbmessgerät der Firma Datacolor, Typ Spectraflash 450 vermessen.

Im Anschluss daran wurde jede gefärbte Strähne 6 manuellen Haarwäschen unterzogen. Für jede Haarwäsche wurde die Strähne angefeuchtet, dann wurde ein handelsübliches Shampoo (Schwarzkopf, Schauma 7 Kräuter) 25 Sekunden lang in die Strähne einmassiert (0,25 g Shampoo pro Gramm Haar). Im Anschluss daran wurde die Strähne 30 Sekunden lang mit lauwarmem Leitungswasser ausgewaschen und getrocknet.

Nach Abschluss der 6 Haarwäschen wurde jede Strähne erneut farbmetrisch vermessen.

Der für die Beurteilung der Waschechtheit herangezogene dE-Wert ergibt sich aus den gemessenen L*a*b*-Farbmesswerten wie folgt:

$$dE = [ (L_i - L_0)^2 + (a_i - a_0)^2 + (b_i - b_0)]^{1/2}$$

$L_0$, $a_0$ und $b_0$ = Messwerte der gefärbten Strähne vor dem Waschen
$L_i$, $a_i$ und $b_i$ = Messwerte der gefärbten Strähne nach 6 Haarwäschen

| | | Verfahren | | 0 Haarwäschen | | | 6 Haarwäschen | | | |
|---|---|---|---|---|---|---|---|---|---|---|
| Bsp. | | (V) | (F) | $L_0$ | $a_0$ | $b_0$ | $L_i$ | $a_i$ | $b_i$ | dE |
| 1 | Vergleich | --- | (F) | 47,18 | 7,11 | 19,31 | 33,05 | 25,50 | 12,02 | 24,31 |
| 2 | Erfindung | (V) | (F) | 34,44 | 48,44 | 15,84 | 36,04 | 43,81 | 12,79 | 5,77 |

Bei Beispiel 1 handelt es sich um das Vergleichsbeispiel, bei dem das Färbemittel (F) ohne Durchführung einer Vorbehandlung appliziert wurde.

Beispiel 2 ist das erfindungsgemäße Beispiel mit sukzessiver Anwendung von Vorbehandlungsmittel (V) und Färbemittel (F).

Bei Anwendung des erfindungsgemäßen Verfahrens wurde ein stark verringerter dE-Wert und demzufolge eine stark verbesserte Waschechtheit beobachtet.

## Patentansprüche

1. Verfahren zum Färben von keratinischen Fasern, insbesondere menschlichen Haaren, umfassend die folgenden Schritte in der angegebenen Reihenfolge:

   (1) Applizieren eines Vorbehandlungsmittels (V) auf die keratinischen Fasern,
   (2) Einwirken des in Schritt (1) applizierten Vorbehandlungsmittels auf die keratinischen Fasern für einen Zeitraum von 2 bis 45 Minuten,
   (3) Ausspülen des Vorbehandlungsmittels mit Wasser,
   (4) Applizieren eines Färbemittels (F) auf die keratinischen Fasern,
   (5) Einwirken des in Schritt (4) applizierten Färbemittels auf die keratinischen Fasern für einen Zeitraum von 15 Sekunden bis 45 Minuten, und
   (6) Ausspülen des Färbemittels mit Wasser,

   wobei

   - das Vorbehandlungsmittel in einem kosmetischen Träger
   (V-1) mindestens ein Oxidationsmittel enthält, und
   - das Färbemittel in einem kosmetischen Träger

     (F-1) mindestens ein aminofunktionalisiertes Silikonpolymer und
     (F-2) mindestens ein Pigment enthält,

   **dadurch gekennzeichnet, dass** zwischen den Schritten (3) und (4) ein Zeitraum von maximal 3 Stunden liegt.

2. Verfahren nach Anspruch 1, **dadurch gekennzeichnet, dass** das Vorbehandlungmittel (V) mindestens ein Oxidationsmittel (V-1) aus der Gruppe aus Wasserstoffperoxid, Ammoniumperoxodisulfat, Kaliumperoxodisulfat und Natriumperoxodisulfat enthält.

3. Verfahren nach einem der Ansprüche 1 bis 2, **dadurch gekennzeichnet, dass** das Vorbehandlungsmittel (V) enthält

   (V-1) Wasserstoffperoxid und
   (V-2) mindestens ein Persulfat aus der Gruppe aus Ammoniumperoxodisulfat, Kaliumperoxodisulfat und Natriumperoxodisulfat.

4. Verfahren nach einem der Ansprüche 1 bis 3, **dadurch gekennzeichnet, dass** das Vorbehandlungsmittel (V) - bezogen auf das Gesamtgewicht des Vorbehandlungsmittels - (V-1) 0,1 bis 12,0 Gew.-% bevorzugt 0,5 bis 10,0 Gew.-%, weiter bevorzugt 1,5 bis 8,0 Gew.-% und ganz besonders bevorzugt 3,0 bis 6,0 Gew.-% Wasserstoffperoxid enthält.

5. Verfahren nach einem der Ansprüche 1 bis 4, **dadurch gekennzeichnet, dass** das Vorbehandlungsmittel (V) - bezogen auf das Gesamtgewicht des Vorbehandlungsmittels - (V-2) ein oder mehrere Persulfate aus der Gruppe aus Ammoniumperoxodisulfat, Kaliumperoxodisulfat und Natriumperoxodisulfat in einer Gesamtmenge von 5,0 bis 30,0 Gew.-%, bevorzugt von 8,0 bis 27,0 Gew.-%, weiter bevorzugt von 11,0 bis 24,0 Gew.-% und ganz besonders von 14,0 bis 21,0 Gew.-% enthält.

6. Verfahren nach einem der Ansprüche 1 bis 5, **dadurch gekennzeichnet, dass** das Färbemittel (F) mindestens ein aminofunktionalisiertes Silikonpolymer (F-1) mit mindestens einer sekundären Aminogruppe enthält.

7. Verfahren nach einem der Ansprüche 1 bis 6, **dadurch gekennzeichnet, dass** das Färbemittel (F) mindestens ein aminofunktionalisiertes Silikonpolymer (F-1) enthält, das mindestens eine Struktureinheit der Formel (Si-Amino) umfasst,

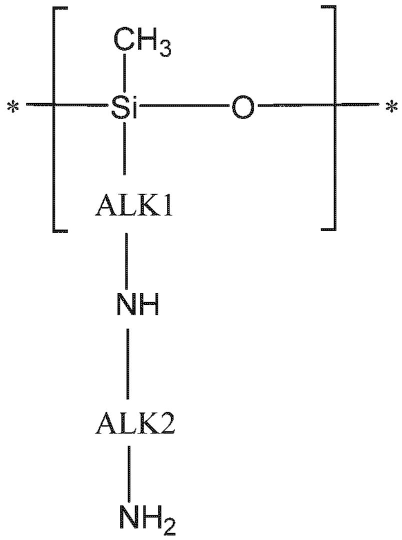

(Si-Amino)

wobei

ALK1 und ALK2 unabhängig voneinander für eine lineare oder verzweigte, zweiwertige $C_1$-$C_{20}$-Alkylengruppe stehen.

8. Verfahren nach einem der Ansprüche 1 bis 7, **dadurch gekennzeichnet, dass** das Färbemittel (F) mindestens ein aminofunktionalisiertes Silikonpolymer (F-1) enthält, das Struktureinheiten der Formel (Si-I) und der Formel (Si-II) umfasst

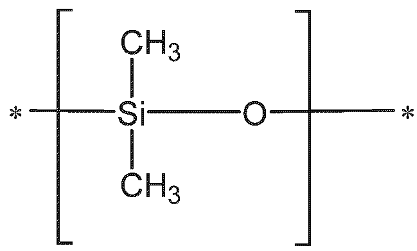

(Si-I)

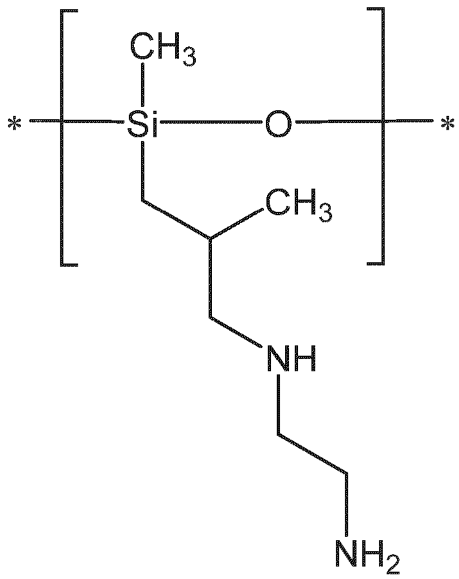

(Si-II).

9. Verfahren nach einem der Ansprüche 1 bis 8, **dadurch gekennzeichnet, dass** das Färbemittel (F) - bezogen auf das Gesamtgewicht des Färbemittels - ein oder mehrere aminofunktionalisierte Silikonpolymere (F-1) in einer Gesamtmenge von 0,1 bis 8,0 Gew.-%, bevorzugt 0,2 bis 5,0 Gew.-%, weiter bevorzugt von 0,3 bis 3,0 Gew.-% und ganz besonders bevorzugt von 0,4 bis 2,5 Gew.-% enthält.

10. Verfahren nach einem der Ansprüche 1 bis 9, **dadurch gekennzeichnet, dass** das Färbemittel (F) mindestens anorganisches Pigment (F-2) enthält, das bevorzugt ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

11. Verfahren nach einem der Ansprüche 1 bis 10, **dadurch gekennzeichnet, dass** das Färbemittel (F) mindestens ein organisches Pigment (F-2) enthält, das bevorzugt ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

12. Verfahren nach einem der Ansprüche 1 bis 11, **dadurch gekennzeichnet, dass** das Färbemittel (F) - bezogen auf das Gesamtgewicht des Färbemittels - ein oder mehrere Pigmente (F-2) in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthält.

13. Verfahren nach einem der Ansprüche 1 bis 12, **dadurch gekennzeichnet, dass** die Gesamtmenge der im Färbemittel (F) enthaltenen direktziehenden Farbstoffe - bezogen auf das Gesamtgewicht des Färbemittels (F) - bei einem Wert unterhalb von 0,1 Gew.-%, bevorzugt unterhalb von 0,05 Gew.-%, weiter bevorzugt unterhalb von 0,01 Gew.-% und ganz besonders bevorzugt unterhalb von 0,001 Gew-% liegt.

14. Verfahren nach einem der Ansprüche 1 bis 13, umfassend die folgenden Schritte in der angegebenen Reihenfolge:

    (1) Applizieren des Vorbehandlungsmittels (V) auf die keratinischen Fasern,
    (2) Einwirken des in Schritt (1) applizierten Vorbehandlungsmittels auf die keratinischen Fasern für einen Zeitraum von 2 bis 30 Minuten und bevorzugt von 2 bis 20 Minuten,
    (3) Ausspülen des Vorbehandlungsmittels mit Wasser,
    (4) Applizieren des Färbemittels (F) auf die keratinischen Fasern,
    (5) Einwirken des in Schritt (4) applizierten Färbemittels auf die keratinischen Fasern für einen Zeitraum von 30 Sekunden bis 30 Minuten und bevorzugt von 1 bis 15 Minuten, und
    (6) Ausspülen des Färbemittels mit Wasser.

## Claims

1. Method for dyeing keratin fibers, in particular human hair, comprising the following steps in the order indicated:

    (1) applying a pretreatment agent (V) to the keratin fibers,
    (2) allowing the pretreatment agent applied in step (1) to act on the keratin fibers for a period of 2 to 45 minutes,
    (3) rinsing off the pretreatment agent with water,
    (4) applying a coloring agent (F) to the keratin fibers,
    (5) allowing the coloring agent applied in step (4) to act on the keratin fibers for a period of 15 seconds to 45 minutes, and
    (6) rinsing the dye agent with water,

    wherein

    - the pretreatment agent in a cosmetic carrier
    (V-1), and
    - the coloring agent in a cosmetic carrier

        (F-1) contains at least one amino-functionalized silicone polymer, and
        (F-2) at least one pigment,

    **characterized in that** there is a period of no more than 3 hours between steps (3) and (4).

2. Method according to claim 1, **characterized in that** the pretreatment agent (V) contains at least one oxidizing agent (V-1) from the group consisting of hydrogen peroxide, ammonium peroxodisulfate, potassium peroxodisulfate, and sodium peroxodisulfate.

**3.** Method according to one of claims 1 to 2, **characterized in that** the pretreatment agent (V) contains

(V-1) hydrogen peroxide and
(V-2) at least one persulfate from the group consisting of ammonium peroxodisulfate, potassium peroxodisulfate, and sodium peroxodisulfate.

**4.** Method according to one of claims 1 to 3, **characterized in that** the pretreatment agent (V) contains - based on the total weight of the pretreatment agent -
(V-1) 0.1 to 12.0 wt.%, preferably 0.5 to 10.0 wt.%, more preferably 1.5 to 8.0 wt.%, and most preferably 3.0 to 6.0 wt.% hydrogen peroxide.

**5.** Method according to one of claims 1 to 4, **characterized in that** the pretreatment agent (V) - based on the total weight of the pretreatment agent -
(V-2) contains one or more persulfates from the group consisting of ammonium peroxodisulfate, potassium peroxodisulfate, and sodium peroxodisulfate in a total amount of 5.0 to 30.0 wt.%, preferably 8.0 to 27.0 wt.%, more preferably 11.0 to 24.0 wt.%, and most preferably 14.0 to 21.0 wt. %.

**6.** Method according to one of claims 1 to 5, **characterized in that** the dye (F) contains at least one amino-functionalized silicone polymer (F-1) with at least one secondary amino group.

**7.** Method according to one of claims 1 to 6, **characterized in that** the dye (F) contains at least one amino-functionalized silicone polymer (F-1) comprising at least one structural unit of the formula (Si-amino)

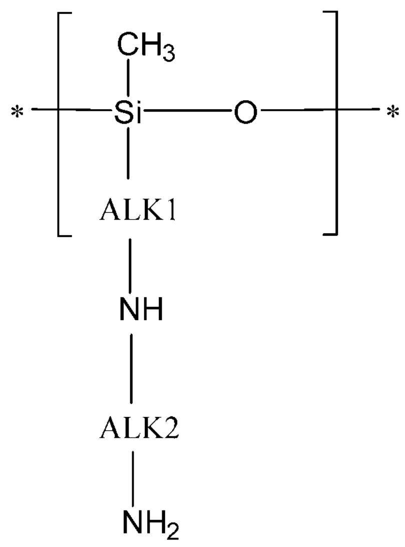

(Si-amino)

where

ALK1 and ALK2 independently represent a linear or branched, divalent $C_1$-$C_{20}$alkylene group.

**8.** Method according to one of claims 1 to 7, **characterized in that** the dye (F) contains at least one amino-functionalized silicone polymer (F-1) comprising structural units of formula (Si-I) and formula (Si-II)

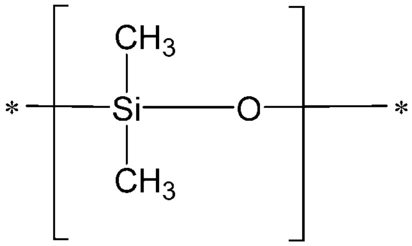

(Si-I)

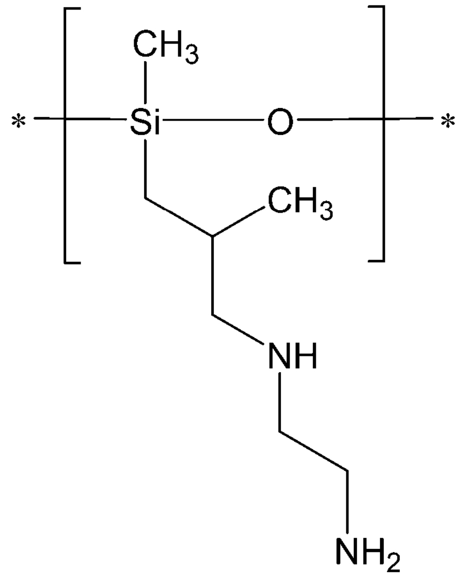

(Si-II).

**9.** Method according to one of claims 1 to 8, **characterized in that** the dye (F) contains, based on the total weight of the dye, one or more amino-functionalized silicone polymers (F-1) in a total amount of 0.1 to 8.0 wt.%, preferably 0.2 to 5.0 wt.%, more preferably from 0.3 to 3.0 wt.%, and most preferably from 0.4 to 2.5 wt.%.

**10.** Method according to one of claims 1 to 9, **characterized in that** the coloring agent (F) contains at least inorganic pigment (F-2), which is preferably selected from the group of colored metal oxides, metal hydroxides, metal oxide hydrates, silicates, metal sulfides, complex metal cyanides, metal sulfates, bronze pigments, and/or colored pigments based on mica or glimmer, which are coated with at least one metal oxide and/or one metal oxychloride.

**11.** Method according to one of claims 1 to 10, **characterized in that** the dye (F) contains at least one organic pigment (F-2) which is preferably selected from the group consisting of carmine, quinacridone, phthalocyanine, sorghum, blue pigments with the Color Index numbers CI 42090, CI 69800, CI 69825, CI 73000, CI 74100, CI 74160, yellow pigments with the Color Index numbers CI 11680, CI 11710, CI 15985, CI 19140, CI 20040, CI 21100, CI 21108, CI 47000, CI 47005, green pigments with Color Index numbers CI 61565, CI 61570, CI 74260, orange pigments with Color Index numbers CI 11725, CI 15510, CI 45370, CI 71105, red pigments with Color Index numbers CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915, and/or CI 75470.

**12.** Method according to one of claims 1 to 11, **characterized in that** the dye (F) contains - based on the total weight of the dye - one or more pigments (F-2) in a total amount of 0.01 to 10.0 wt.%, preferably 0.1 to 5.0 wt.%, more preferably from 0.2 to 2.5 wt.%, and most preferably from 0.25 to 1.5 wt.%.

**13.** Method according to one of claims 1 to 12, **characterized in that** the total amount of direct dyes contained in the dyeing agent (F) - based on the total weight of the dyeing agent (F) - is less than 0.1 wt.%, preferably less than 0.05 wt.%, more preferably less than 0.01 wt.%, and most preferably less than 0.001 wt.%.

**14.** Method according to one of claims 1 to 13, comprising the following steps in the order indicated:

(1) Applying the pretreatment agent (V) to the keratin fibers,
(2) allowing the pretreatment agent applied in step (1) to act on the keratin fibers for a period of 2 to 30 minutes, and preferably 2 to 20 minutes,
(3) Rinsing off the pretreatment agent with water,
(4) applying the dye (F) to the keratin fibers,
(5) Allowing the dye applied in step (4) to act on the keratin fibers for a period of 30 seconds to 30 minutes, and preferably 1 to 15 minutes, and
(6) Rinsing the dye off with water.

## Revendications

1. Procédé de teinture de fibres kératiniques, en particulier de cheveux humains, comprenant les étapes suivantes dans l'ordre indiqué :

   (1) application d'un agent de prétraitement (V) sur les fibres kératiniques,
   (2) laisser agir l'agent de prétraitement appliqué à l'étape (1) sur les fibres kératiniques pendant une durée de 2 à 45 minutes,
   (3) rinçage de l'agent de prétraitement à l'eau,
   (4) application d'un colorant (F) sur les fibres kératiniques,
   (5) laisser agir le colorant appliqué à l'étape (4) sur les fibres kératiniques pendant une durée comprise entre 15 secondes et 45 minutes, et
   (6) rinçage du colorant à l'eau,

   où

   - l'agent de prétraitement dans un support cosmétique
   (V-1), et
   - le colorant contient dans un support cosmétique

     (F-1) au moins un polymère de silicone à fonction amine et
     (F-2) au moins un pigment,

   **caractérisé en ce qu'**il s'écoule un intervalle de temps maximal de 3 heures entre les étapes (3) et (4).

2. Procédé selon la revendication 1, **caractérisé en ce que** l'agent de prétraitement (V) contient au moins un agent oxydant (V-1) choisi dans le groupe constitué par le peroxyde d'hydrogène, le peroxodisulfate d'ammonium, le peroxodisulfate de potassium et le peroxodisulfate de sodium.

3. Procédé selon l'une des revendications 1 à 2, **caractérisé en ce que** l'agent de prétraitement (V) contient

   (V-1) du peroxyde d'hydrogène et
   (V-2) au moins un persulfate choisi parmi le peroxodisulfate d'ammonium, le peroxodisulfate de potassium et le peroxodisulfate de sodium.

4. Procédé selon l'une des revendications 1 à 3, **caractérisé en ce que** l'agent de prétraitement (V) contient, par rapport au poids total de l'agent de prétraitement
   (V-1) 0,1 à 12,0 % en poids, de préférence 0,5 à 10,0 % en poids, de préférence encore 1,5 à 8,0 % en poids et de manière particulièrement préférée 3,0 à 6,0 % en poids de peroxyde d'hydrogène.

5. Procédé selon l'une des revendications 1 à 4, **caractérisé en ce que** l'agent de prétraitement (V) contient, par rapport au poids total de l'agent de prétraitement
   (V-2) un ou plusieurs persulfates choisis dans le groupe constitué par le peroxodisulfate d'ammonium, de peroxodisulfate de potassium et de peroxodisulfate de sodium, en une quantité totale de 5,0 à 30,0 % en poids, de préférence de 8,0 à 27,0 % en poids, de manière encore plus préférée de 11,0 à 24,0 % en poids et de manière tout à fait particulière de 14,0 à 21,0 % en poids.

6. Procédé selon l'une des revendications 1 à 5, **caractérisé en ce que** le colorant (F) contient au moins un polymère de silicone à fonction amino (F-1) avec au moins un groupe amino secondaire.

7. Procédé selon l'une des revendications 1 à 6, **caractérisé en ce que** le colorant (F) contient au moins un polymère de silicone à fonction amino (F-1) qui comprend au moins une unité structurale de formule (Si-amino)

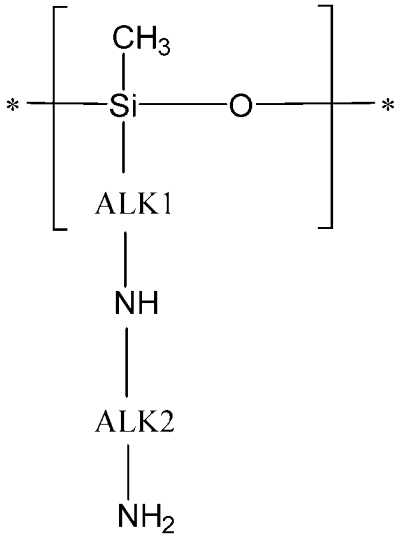

(Si-amino)

où

ALK1 et ALK2 représentent indépendamment l'un de l'autre un groupe alkylène divalent linéaire ou ramifié en $C_1$-$C_{20}$.

**8.** Procédé selon l'une des revendications 1 à 7, **caractérisé en ce que** le colorant (F) contient au moins un polymère de silicone à fonction amino (F-1) qui comprend des motifs structuraux de formule (Si-I) et de formule (Si-II)

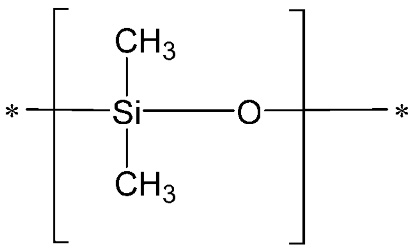

(Si-I)

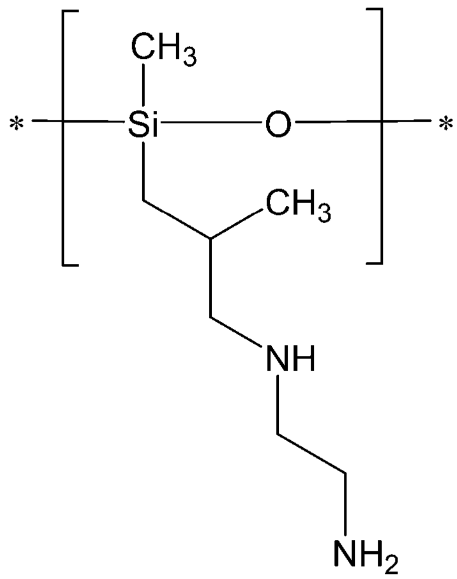

(Si-II).

**9.** Procédé selon l'une des revendications 1 à 8, **caractérisé en ce que** le colorant (F) contient, par rapport au poids total du colorant, un ou plusieurs polymères de silicone à fonction amine (F-1) en une quantité totale de 0,1 à 8,0 % en poids, de préférence de 0,2 à 5,0 % en poids, de préférence de 0,3 à 3,0 % en poids et de manière particulièrement préférée de 0,4 à 2,5 % en poids.

**10.** Procédé selon l'une des revendications 1 à 9, **caractérisé en ce que** le colorant (F) contient au moins un pigment inorganique (F-2) qui est de préférence choisi dans le groupe des oxydes métalliques colorés, des hydroxydes métalliques, des hydrates d'oxydes métalliques, des silicates, sulfures métalliques, cyanures métalliques complexes, sulfates métalliques, pigments de bronze et/ou de pigments colorés à base de mica ou de micacé, qui sont recouverts d'au moins un oxyde métallique et/ou un oxychlorure métallique.

**11.** Procédé selon l'une des revendications 1 à 10, **caractérisé en ce que** le colorant (F) contient au moins un pigment organique (F-2) choisi de préférence dans le groupe constitué par le carmin, la quinacridone, la phtalocyanine, le sorgho, les pigments bleus ayant les numéros d'index de couleur CI 42090, CI 69800, CI 69825, CI 73000, CI 74100, CI 74160, des pigments jaunes ayant les numéros d'index de couleur CI 11680, CI 11710, CI 15985, CI 19140, CI 20040, CI 21100, CI 21108, CI 47000, CI 47005, pigments verts portant les numéros d'index de couleur CI 61565, CI 61570, CI 74260, pigments orange portant les numéros d'index de couleur CI 11725, CI 15510, CI 45370, CI 71105, pigments rouges avec les numéros d'index de couleur CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 et/ou CI 75470.

**12.** Procédé selon l'une des revendications 1 à 11, **caractérisé en ce que** le colorant (F) contient, par rapport au poids total du colorant, un ou plusieurs pigments (F-2) en une quantité totale de 0,01 à 10,0 % en poids, de préférence de 0,1 à 5,0 % en poids, de préférence de 0,2 à 2,5 % en poids et de manière particulièrement préférée de 0,25 à 1,5 % en poids.

**13.** Procédé selon l'une des revendications 1 à 12, **caractérisé en ce que** la quantité totale des colorants directs contenus dans le colorant (F) - par rapport au poids total du colorant (F) - est inférieure à 0,1 % en poids, de préférence inférieure à 0,05 % en poids, de préférence encore inférieure à 0,01 % en poids et de manière particulièrement préférée inférieure à 0,001 % en poids.

**14.** Procédé selon l'une des revendications 1 à 13, comprenant les étapes suivantes dans l'ordre indiqué :

(1) application de l'agent de prétraitement (V) sur les fibres kératiniques,
(2) laisser agir l'agent de prétraitement appliqué à l'étape (1) sur les fibres kératiniques pendant une durée de 2 à 30 minutes, de préférence de 2 à 20 minutes,
(3) rinçage de l'agent de prétraitement à l'eau,
(4) application du colorant (F) sur les fibres kératiniques,
(5) laisser agir le colorant appliqué à l'étape (4) sur les fibres kératiniques pendant une durée comprise entre 30 secondes et 30 minutes, et de préférence entre 1 et 15 minutes, et
(6) rinçage du colorant à l'eau.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2017108828 A1
- US 2013149358 A1
- DE 102014218006 A1